# EUROPEAN PATENT SPECIFICATION

(11) **EP 4 083 031 B1**
(45) Date of publication and mention of the grant of the patent: **22.04.2026**
(21) Application number: 20904375.1
(22) Date of filing: 22.12.2020
(51) Int. Cl.: C07D 401/04, A61P 35/00, A61K 31/47

(54) **QUINOLINE COMPOUND AND APPLICATION THEREOF AS FGFR4 KINASE INHIBITOR**
CHINOLINVERBINDUNG UND ANWENDUNG DAVON ALS FGFR4-KINASEHEMMER
COMPOSÉ DE QUINOLÉINE ET SON APPLICATION ENT TANT QU'INHIBITEUR DE LA FGFR4 KINASE

(30) Priority: 23.12.2019 CN 201911337586
(43) Date of publication of application: 02.11.2022
(73) Proprietor: Skyrun Pharma Co., Ltd., Jiangsu 210046 (CN)
(72) Inventor: YAN, Libo, Nanjing, Jiangsu 210046 (CN); HU, Shihe, Nanjing, Jiangsu 210046 (CN); WANG, Ya, Nanjing, Jiangsu 210046 (CN); LIU, Yu, Nanjing, Jiangsu 210046 (CN)
(74) Representative: Novagraaf Technologies
(86) International application number: PCT/CN2020/138248
(87) International publication number: WO 2021/129596

(56) References cited:
- WO-A1-2009/097325
- WO-A1-2015/061572
- CN-A- 101 573 340
- CN-A- 101 611 011
- CN-A- 104 011 025
- LIU FENG-TAO ET AL: "Recent advance in the development of novel, selective and potent FGFR inhibitors", EUROPEAN JOURNAL OF MEDICINAL CHEMISTRY, ELSEVIER, AMSTERDAM, NL, vol. 186, 16 November 2019 (2019-11-16), XP086035820, ISSN: 0223-5234, [retrieved on 20191116], DOI: 10.1016/J.EJMECH.2019.111884

## Description

### Technical Field

The present disclosure belongs to the technical field of medicines, and specifically relates to a class of quinoline compounds and use thereof in preparation of a drug for preventing and/or treating fibroblast growth factor receptor 4 (FGFR4) overexpression-mediated diseases in organisms and diseases associated with angiogenesis or cancer metastasis.

### Background

Protein receptor tyrosine kinases (RTKs) play an important role in cell signal transduction pathways, it transmits an extracellular signal into cells, and regulates physiological activities, such as proliferation, differentiation, growth and apoptosis, of tumor cells by downstream transduction. RTKs are closely related to the occurrence and development of tumors, therefore, its family members become mainstream targets in the development of anti-tumor drugs. At present, nearly 100 tyrosine kinase inhibitors are marketed or enter clinical trials, most of which are reversible inhibitors. These inhibitors have the following disadvantages: 1) poor selectivity; 2) short reaction time; and 3) easy to produce drug resistance. Based on the above reasons, the research and development of irreversible kinase inhibitors become a hot direction. The irreversible kinase inhibitor is based on a reversible inhibitor, a Michael acceptor fragment is linked to an appropriate position, and the electrophilic fragment may perform an electrophilic reaction with a cysteine residue near an adenosine triphosphate (ATP) binding domain to form a covalent bond, thereby the action is achieved by a way of permanent inactivation, and this way makes its effect more intense and long-lasting. At the same time, there is no competition between the irreversible kinase inhibitors and ATP and kinase binding, which also reduces the possibility of kinase mutation and alleviates or avoids the generation of the drug resistance, so the selectivity is higher.

FGFR is a receptor-type protein tyrosine kinase, and there are four types in total: FGFR1, FGFR2, FGFR3 and FGFR4, they play a key role in maintaining the growth, proliferation, apoptosis and migration and the like of cells. It is indicated from researches that FGFR4 is highly expressed in a plurality of cancer cells, and has the effects of regulating the cell proliferation and anti-apoptosis. It may be used as an important target for aggressive cancers, while FGFR4 is gene-knocked out, the cancer cell proliferation may be effectively reduced, and the apoptosis of the cancer cells is promoted, and at the same time, selective action on FGFR4 may avoid the toxic and side effects caused by acting on several other subtypes such as FGFR1~3. The mutation and aberrant activity of FGFR4 in various cancers, especially a liver cancer, makes it to become a drug target for treatment of FGFR4-positive cancers.

At present, a plurality of FGFR4 kinase inhibitors enters clinical trials for the treatment of the various cancers, but no FGFR4 kinase irreversible inhibitor is marketed yet. Therefore, the development of a new FGFR4 kinase irreversible inhibitor with high inhibitory activity and excellent pharmacokinetic properties already becomes a key to the development of a new-type antitumor drug.

The present invention relates to quinoline compound and use thereof, relevant technologies are known from LIU FENG-TAO ET AL: "Recent advance in the development of novel, selective and potent FGFR inhibitors" (EUROPEAN JOURNAL OF MEDICINAL CHEMISTRY, ELSEVIER, AMSTERDAM, NL, vol. 186, 16 November 2019, XP086035820) and WO 2015/061572 A1.

### Summary

In view of deficiencies of the above existing technology, a purpose of the present disclosure is to provide a quinoline compound, the compound has good inhibitory activity on an FGFR4 kinase, may be used as an FGFR4 irreversible inhibitor, and provides the possibility for treatment of FGFR4-mediated diseases.

In order to achieve the above purpose of the present disclosure, the present disclosure adopts the following technical means.

A compound of Formula I or a pharmaceutically acceptable salt, crystal form, stereoisomer, tautomer, hydrate or solvate thereof:

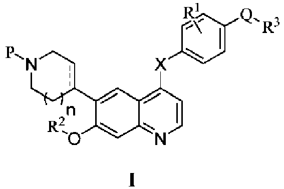

In Formula I:
X is selected from -CR⁴R⁵-, -NR⁴-, O or S, herein R⁴ and R⁵ each independently represent hydrogen, deuterium, halogen, alkyl, aryl or Het;
Q is selected from -NR⁶CONR⁷-, -CONR⁶-, -NR⁶CO-, -NR⁶SO₂NR⁷-, -SO₂NR⁶-, NR⁶SO₂-, -NR⁶-, -NR⁶(CH₂)mN-, -NR⁶(CH₂)mO- or -CR⁶R⁷, herein m=1, 2, 3, 4 or 5, R⁶, and R⁷ each independently represent hydrogen, deuterium, alkyl, aryl or Het;
a dotted line represents that a single-band or a double-bond may be present;
n=0, 1, 2 or 3;
R¹ is selected from hydrogen, halogen, hydroxyl, alkoxy, alkyl, aryl or Het;
R² is selected from hydrogen, halogen, hydroxyl, alkoxy, alkyl, aryl or Het;
R³ is selected from hydrogen, halogen, hydroxyl, alkoxy, alkyl, aryl or Het;
P is selected from the following structures: 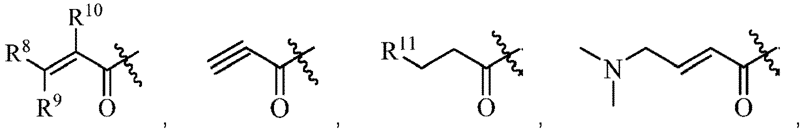 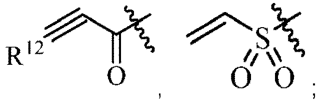
herein, R¹¹ is a leaving group or an activated ester, and the leaving group is a halogen; R⁸, R⁹, R¹⁰, and R¹² are each independently selected from hydrogen, halogen, hydroxyl, alkoxy, alkyl, aryl or Het;
the alkyl is a straight-chain or branched-chain saturated hydrocarbyl of 1-6 carbon atoms, a cyclic saturated hydrocarbyl of 3-6 carbon atoms, or a cyclic saturated hydrocarbyl of 3-6 carbon atoms linked with a straight-chain or branched-chain saturated hydrocarbyl of 1-6 carbon atoms;
in the above groups, the aryl is a carbocyclic ring selected from phenyl, naphthyl, acenaphthyl or tetrahydronaphthyl, each of which is optionally substituted by 1, 2 or 3 substituents, and each substituent is independently selected from hydrogen, alkyl, cyano, halogen, nitro, haloalkyl, hydroxyl, mercapto, alkoxy, alkylthio, alkoxyalkyl, aralkyl, diarylalkyl, aryl or Het;
Het is a monocyclic heterocycle selected from piperidinyl, pyrrolyl, pyrazolyl, imidazolyl, furyl, thienyl, oxazolyl, isoxazolyl, thiazolyl, isothiazolyl, pyridyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxenyl or benzo[1,3]dioxolyl; and each monocyclic or bicyclic heterocycle is optionally substituted with 1, 2 or 3 substituents, and each substituent is independently selected from halogen, haloalkyl, hydroxy, alkyl or alkoxy;
the halogen is selected from fluorine, chlorine, bromine or iodine.

Further, in Formula I:
X is -NR⁴- or O, herein R⁴ is selected from hydrogen, deuterium, halogen, alkyl, aryl or Het;
Q is selected from -NR⁶CONR⁷-, -CONR⁶-, -NR⁶CO-, -NR⁶SO₂NR⁷-, -SO₂NR⁶- or -NR⁶SO₂-, herein R⁶, and R⁷ independently represent hydrogen, deuterium, alkyl, aryl or Het;
a dotted line indicates that a single-bond or a double-bond may be present;
n=0, 1, 2 or 3;
R¹ is selected from hydrogen, halogen or alkyl;
R² is selected from hydrogen, alkyl or aryl;
R³ is selected from alkoxy, alkyl, aryl or Het.

Further, in Formula I:
X is O;
Q is selected from -NR⁶CONR⁷-, -CONR⁶- or -NR⁶CO-, herein R⁶, and R⁷ each independently represent hydrogen, deuterium, alkyl, aryl or Het;
A dotted line indicates that a single-bond or a double-bond may be present;
n=0, 1, 2 or 3;
R¹ is selected from hydrogen, halogen or alkyl;
R² is alkyl or aryl;
R³ is selected from alkyl, aryl or Het.

Further, in Formula I:
X is O;
Q is selected from -NR⁶CONR⁷-, -CONR⁶- or -NR⁶CO-, herein R⁶, and R⁷ each independently represent hydrogen, deuterium, alkyl, aryl or Het;
A dotted line indicates that a single-bond or a double-bond may be present;
n=0 or 1;
R¹ is hydrogen or halogen;
R² is alkyl or aryl;
R³ is selected from alkyl, aryl or Het;
P is selected from the following structures: 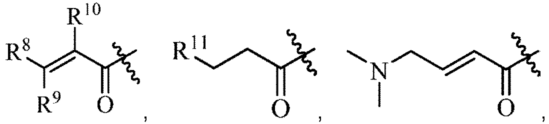
herein, R¹¹ is a leaving group or an activated ester, and the leaving group is a halogen; R⁸, R⁹ and R¹⁰ are each independently selected from hydrogen or alkyl.

Further, the pharmaceutically acceptable salt includes an acid addition salt formed by the compound of Formula I with the following acids: hydrochloric acid, hydrobromic acid, sulfuric acid, phosphoric acid, methanesulfonic acid, benzenesulfonic acid, p-toluenesulfonic acid, naphthalenesulfonic acid, citric acid, tartaric acid, lactic acid, pyruvic acid, acetic acid, maleic acid or succinic acid, fumaric acid, salicylic acid, phenylacetic acid, and mandelic acid; and also includes an acidic salt formed by the compound of Formula I with an inorganic base.

Further, the pharmaceutically acceptable salt includes an alkaline metal cation salt, an alkaline earth metal cation salt and an ammonium cation salt.

Further, the compound of Formula I is one of the following compounds:

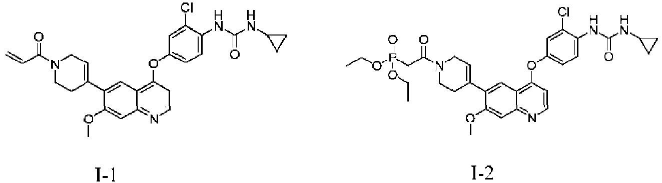

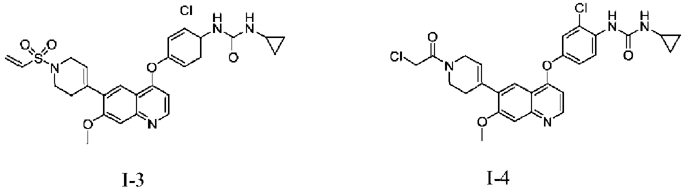

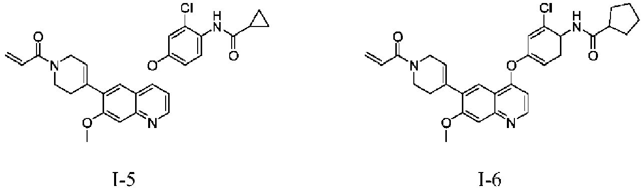

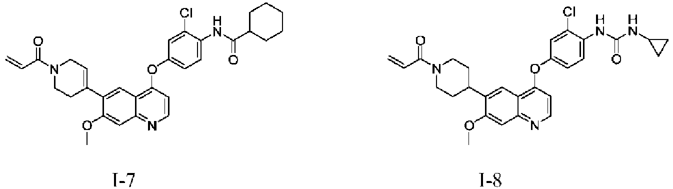

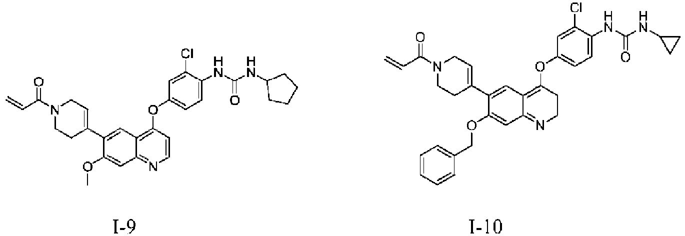

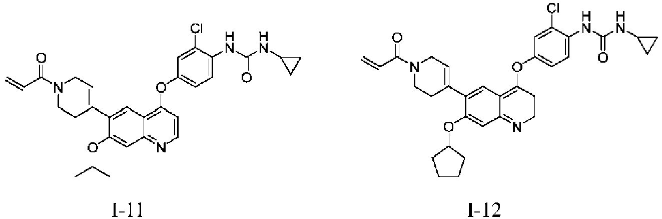

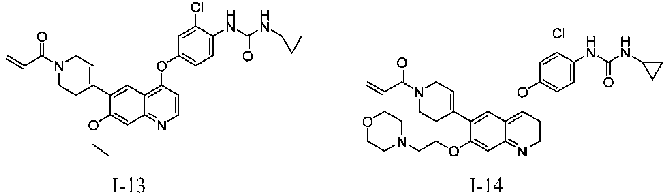

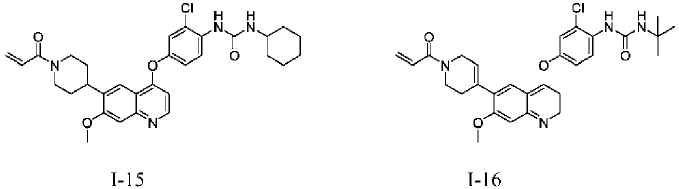

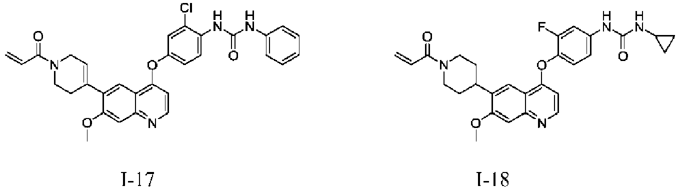

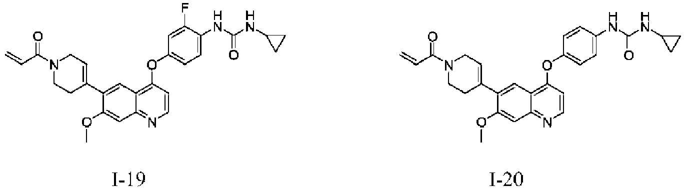

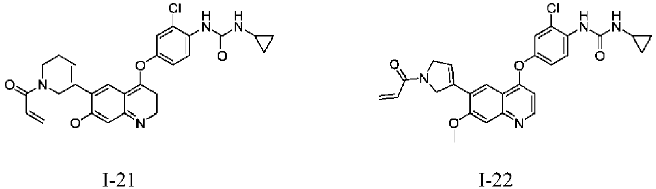

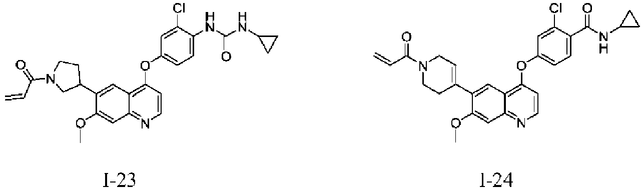

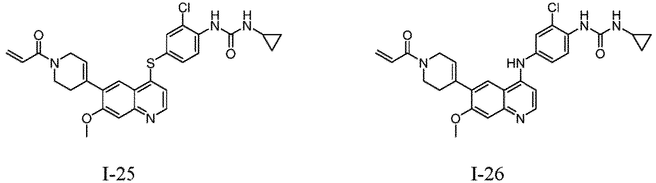

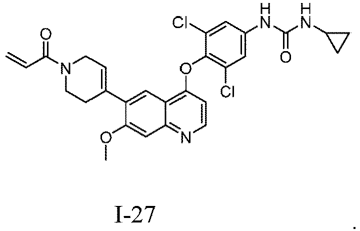

A pharmaceutical composition includes the above compound or the pharmaceutically acceptable salt, crystal form, stereoisomer, tautomer, hydrate or solvate thereof and a pharmaceutically acceptable carrier or excipient.

Use of the above compound in preparation of a drug for preventing and/or treating FGFR4-related diseases.

Further, the FGFR4-related disease is selected from but not limited to hyperlipidemia or cancer; and the cancer includes lung cancer, squamous cell carcinoma, bladder cancer, gastric cancer, ovarian cancer, peritoneal cancer, breast cancer, breast ductal carcinoma, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin lymphoma, colorectal villous adenomas, melanomas, cell tumor or sarcoma, or myelodysplastic syndrome.

Beneficial effect: the compound of Formula I and the pharmaceutically acceptable salt, crystal form, stereoisomer, tautomer, hydrate or solvate thereof provided by the present disclosure serve as the receptor tyrosine kinase inhibitor, especially it has the excellent inhibitory activity to the FGFR4 kinase. Therefore, the above compound may be used to prepare the drug for treatment of clinical symptoms related to FGFR4, such as: the use in preparation of the drug for preventing and/or treating the diseases related to FGFR4-related abnormal cell proliferation, morphological changes and hyperkinesias and the like in the organisms and the drug for the diseases associated with angiogenesis or cancer metastasis.

### Detailed Description of the Embodiments

The present disclosure provides a compound of Formula I:

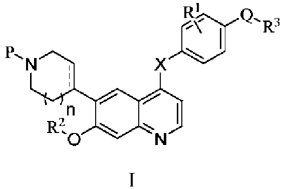

It is indicated from pharmacological test results that the compound has good inhibitory activity to FGFR4, may be used as a new-type FGFR4 irreversible inhibitor, and provides the possibility for the treatment of FGFR4-mediated diseases.

In the present disclosure, the compound of Formula I may also exist in the form of its salt, hydrate, and solvate, and they are converted to the compound of Formula I in vivo. For example, within a scope of the present disclosure, and according to a known process in the field, the compound of the present disclosure is converted into a pharmaceutically acceptable salt form, and they are used in the salt form.

At the same time, the compound of Formula I may also exist in a polycrystalline or amorphous form.

In addition, the compound of Formula I may also exist in a specific geometric or stereoisomeric form. Additional asymmetric carbon atoms may be present in substituents such as an alkyl, and all such isomers and mixtures thereof are included within the scope of the present disclosure.

The pharmaceutical composition involved in the present disclosure includes the compound of Formula I or the pharmaceutically acceptable salt, crystal form, stereoisomer, tautomer, hydrate or solvate thereof and the pharmaceutically acceptable carrier or excipient.

The pharmaceutical composition of the present disclosure may be administered in a variety of known modes, such as orally administration, parenterally administration, administration by inhalation spray or administration by an implanted depot. The pharmaceutical composition of the present disclosure may be administered alone or in combination with other antitumor drugs. An oral composition may be in any orally acceptable dosage forms, including but not limited to tablets, capsules, emulsions, and suspensions, dispersions and solution. The commonly used pharmaceutically acceptable carrier or excipient includes a stabilizer, a diluent, a surfactant, a lubricant, an antioxidant, a binder, a colorant, a filler, an emulsifier and the like.

A sterile injectable composition may be prepared according to a technology known in the field by using a suitable dispersing agent or wetting agent and a suspending agent. The pharmaceutically acceptable carrier and solvent that may be used include water, mannitol, sodium chloride solution and the like.

A topical composition may be prepared into oils, lotions, creams and the like. The carrier for the composition includes vegetable oil or mineral oil, animal fat, high molecular weight alcohol and the like. The pharmaceutically acceptable carrier is a carrier in which an active ingredient is soluble.

The actual dosage level of the active ingredient in the pharmaceutical composition of the present disclosure may be changed to obtain an amount of the active ingredient effective to achieve the desired therapeutic response for a particular patient, composition, and mode of administration, without being toxic to the patient. The dosage level selected depends on a plurality of factors, including the activity of a specific compound of the present disclosure or the salt thereof used, the route of administration, the time of administration, the rate of excretion of the specific composition used, the duration of treatment, and other drugs, compounds and/or materials used in combination in the specific composition used, the age, sex, weight, general health and past medical history of a patient being treated, and similar factors well-known in the medical field.

The present disclosure further provides a use of the compound of Formula I or the pharmaceutically acceptable salt, crystal form, stereoisomer, tautomer, hydrate or solvate thereof in preparation of a drug for preventing and/or treating FGFR4-related diseases, such as: the use in preparation of the drug for preventing and/or treating the diseases related to FGFR4-related abnormal cell proliferation, morphological changes and hyperkinesias and the like in the organisms and the drug for the diseases associated with angiogenesis or cancer metastasis.

The FGFR4-related disease is selected from but not limited to hyperlipidemia or cancer; and the cancer includes lung cancer, squamous cell carcinoma, bladder cancer, gastric cancer, ovarian cancer, peritoneal cancer, breast cancer, breast ductal carcinoma, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin lymphoma, colorectal villous adenomas, melanomas, cell tumor and sarcoma, and myelodysplastic syndrome.

The preparation method for the compound of Formula I of the present disclosure is described below in combination with specific embodiments, but these specific methods do not constitute any limitations to the present disclosure. The compound of the present disclosure may also be conveniently prepared by optionally combining various synthetic methods described in the description or known in the field, and such combinations may be easily performed by those skilled in the art to which the present disclosure belongs.

Starting materials, reaction reagents and the like used in the specific embodiments of the present disclosure are all commercially available. The present disclosure may be prepared in the form of a salt by adopting a salt-forming method commonly used in the field.

### Embodiment 1

### 4-[3-chloro-4-(cyclopropylaminocarbonyl)aminophenoxy]-6-[(1-acryloyl-1,2,3,6-tetrahydropyridine)-4-yl]-7-methoxyquinoline (I-1)

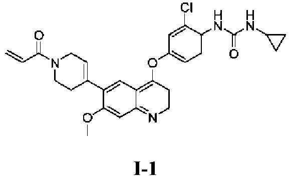

A synthetic route is as follows:

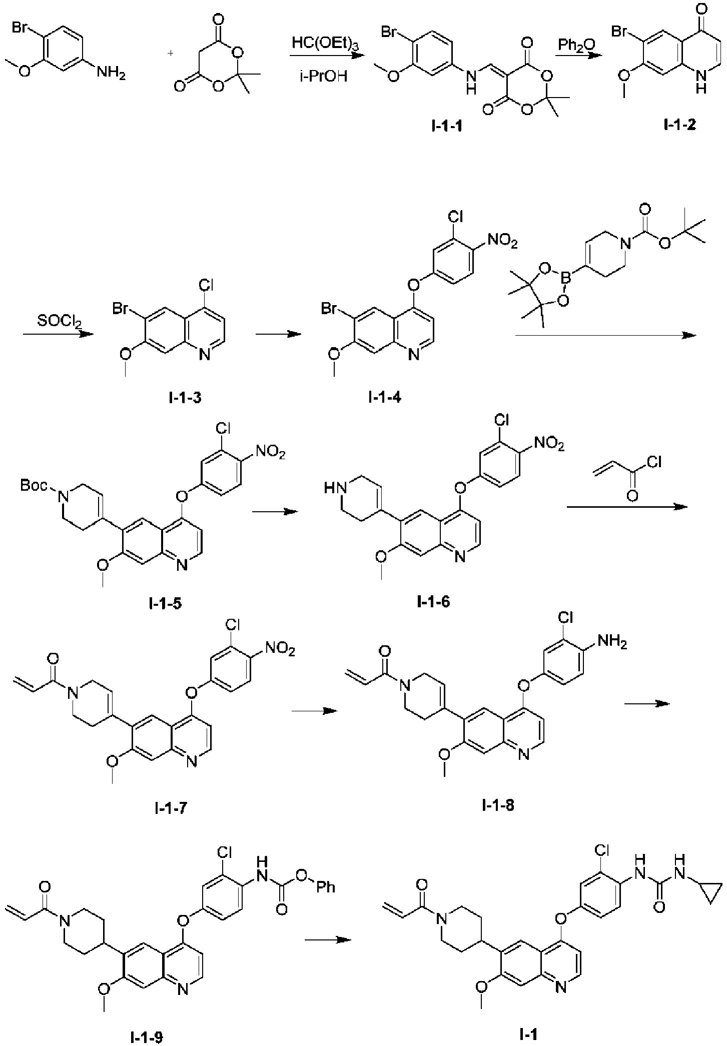

### Synthesis of compound I-1-1:

In a 500 mL single-neck flask, isopropylidene malonate (35.50 g, and 248 mmol), triethyl orthoformate (36.60 g, and 248 mmol) and 300 mL of isopropanol are added, the temperature is raised to 90°C and it is reacted for 2.5 h, then 4-bromo-3-methoxyaniline (25.00 g, and 124 mmol) is added in batches, it continues to react at 90°C for 3 h, the completion of the reaction is monitored by a thin layer chromatography (TLC) (petroleum ether (PE): ethyl acetate (EA)=1:1), reaction solution is cooled to a room temperature, a solid is precipitated and filtered, and a filter cake is washed with ethanol for 3 times, and the filter cake is dried to obtain 40.02 g of a white solid, yield: 91%, and MS m/z: 357.1 [M+H]⁺.

### Synthesis of compound I-1-2:

In a 1000 mL single-neck flask, I-1-1 (30.00 g, and 84 mmol) and 600 mL of diphenyl ether are added, the temperature is raised to 230°C and it is reacted for 0.5 h, the completion of the reaction is monitored by TLC (PE:EA=1:1), reaction solution is cooled to the room temperature, a solid is precipitated, and filtered, a filter cake is washed with ethanol for 3 times, and the filter cake is dried to obtain 18.03 g of a light yellow solid, yield: 84%, MS m/z: 255.1 [M+H]⁺.

### Synthesis of compound I-1-3:

In a 500 mL single-neck flask, 1-1-2 (18.00 g, and 70.8 mmol), 300 mL of thionyl chloride and 1 mL of dimethyl formamide (DMF) are added, the temperature is raised to 90°C and it is reacted for 2 h, the completion of the reaction is monitored by TLC (PE:EA=1:1), reaction solution is cooled to the room temperature, and after the reaction solution is concentrated to dryness, 300 mL of saturated sodium bicarbonate aqueous solution is added in batches under stirring, it is extracted with EA (150 mL×3), an organic phase is dried and concentrated, and a column chromatography (PE:EA=5:1) is performed to obtain 14.60 g of a light yellow solid, yield: 76%, MS m/z: 273.5 [M+H]⁺.

### Synthesis of compound I-1-4:

In a 500 mL single-neck flask, 3-chloro-4-nitrophenol (5.60 g, and 32.1 mmol) and 200 mL of DMF are added, the temperature is reduced to 0°C, sodium hydrogen (1.60 g, and 40.2 mmol) is added in batches, it continues to react at 0°C for 0.5 h, I-1-3 (7.30 g, and 26.8 mmol) is added, the temperature is raised to 160°C and it is reacted for 16 h, the completion of the reaction is monitored by TLC (PE:EA=1:1), reaction solution is cooled to the room temperature, 300 mL of ice water is added to the reaction solution, it is extracted with EA (100 mL×3), an organic phase is dried and concentrated, and a column chromatography (PE:EA=5:1) is performed to obtain 3.41 g of a yellow solid, yield: 31%, MS m/z: 410.2 [M+H]⁺.

### Synthesis of compound I-1-5:

In a 100 mL single-neck flask, I-1-4 (3.40 g, and 8.3 mmol), N-Boc-1,2,5,6-tetrahydropyridine-4-boronic acid pinacol ester (2.80 g, and 9.1 mmol), a potassium carbonate (0.46 g, and 33.2 mmol), 60 mL of dioxane and 20 mL of water are added respectively, and under a nitrogen atmosphere, Pd(dppf)Cl₂ (0.60 g, and 0.8 mmol) is added, a nitrogen gas is replaced for 3 times, the temperature is raised to 110°C and it is reacted for 3 h, the completion of the reaction is monitored by TLC (PE:EA=1:1), reaction solution is cooled to the room temperature, 100 mL of ice water is added to the reaction solution, it is extracted with EA (50 mL×3), an organic phase is dried and concentrated, and a column chromatography (PE:EA=4:1) is performed to obtain 1.50 g of a yellow solid, yield: 35%, MS m/z: 512.1 [M+H]⁺.

### Synthesis of compound I-1-6:

In a 100 mL single-neck flask, 1-1-5 (0.80 g, and 1.6 mmol), 2 mL of trifluoroacetic acid and 15 mL of dichloromethane are added respectively, it is reacted at the room temperature for 1 h, it is detected by liquid chromatography and mass spectrometry (LCMS) that there are no remaining raw materials, and reaction solution is directly concentrated to obtain a crude product, 0.71 g of a deep yellow solid, MS m/z: 412.1 [M+H]⁺.

### Synthesis of compound I-1-7:

In a 100 mL single-neck flask, I-1-6 (0.70 g, and 1.7 mmol), triethylamine (0.50 g, and 5.1 mmol) and 10 mL of dichloromethane are added respectively, the temperature is reduced to 0°C, acryloyl chloride (0.20 g, and 2.0 mmol) is dropwise added, and after the dropwise addition is completed, it is reacted at the room temperature for 2 h, it is detected by LCMS that there are no remaining raw materials, 10 mL of water is added, it is extracted with dichloromethane (50 mL×3), organic phases are combined, concentrated and spin-dried, a column chromatography (PE:EA=3:1) is performed to obtain 0.52 g of a yellow solid, yield: 66%, MS m/z: 466.3 [M+H]⁺.

### Synthesis of compound I-1-8:

In a 100 mL single-neck flask, I-1-7 (0.52 g, and 1.1 mmol), reduced iron powder (0.60 g, and 11.0 mmol), 10 drops of hydrochloric acid and 10 mL of ethanol are added respectively, the temperature is raised to 80°C and it is reacted for 2 h, it is detected by LCMS that there are no remaining raw materials, reaction solution is directly concentrated to obtain a crude product, 10 mL of water is added to it, it is extracted with ethyl acetate (10 mL×3), organic phases are combined, it is washed twice with saturated sodium chloride aqueous solution, and dried with an anhydrous sodium sulfate, and an organic layer is concentrated and spin-dried, to obtain 0.40 g of a crude product for future use, MS m/z: 436.9 [M+H]⁺.

### Synthesis of compound I-1-9:

In a 50 mL single-neck flask, 1-1-8 (0.05 g, and 0.11 mmol), phenyl chloroformate (0.036 g, and 0.22 mmol), 1 mL of N-methylpyrrolidone and 4 mL of acetonitrile are added respectively, the temperature is raised to 70°C and it is reacted for 3 h, it is detected by LCMS that there are no remaining raw materials, 5 mL of water is added to it, it is extracted with ethyl acetate (5 mL×3), organic phases are combined, it is washed twice with saturated sodium chloride aqueous solution, and dried with an anhydrous sodium sulfate, and an organic layer is concentrated and spin-dried, to obtain 0.06 g of a crude product for future use, MS m/z: 559.2 [M+H]⁺.

### Synthesis of compound I-1 Synthesis of:

In a 50 mL single-neck flask, I-1-9 (0.06 g, and 0.11 mmol), cyclopropylamine (0.013 g, and 0.22 mmol), 1 mL of N,N-diisopropylethylamine and 4 mL of tetrahydrofuran are added respectively, the temperature is raised to 65°C and it is reacted for 3 h, it is detected that there are no remaining raw materials, 15 mL of water is added to it, it is extracted with ethyl acetate (15 mL×3), organic phases are combined, it is washed twice with saturated sodium chloride aqueous solution, and dried with an anhydrous sodium sulfate, an organic layer is concentrated and spin-dried, and it is purified by TLC to obtain 0.02 g of a yellow solid, yield: 36%, MS m/z: 519.3 [M+H]⁺.
¹H-NMR(400MHz,DMSO-
d₆)δ(ppm):8.56(d,J=5.4Hz,1H),8.20(d,J=9.0Hz,1H),8.14(s,1H),7.39(s,2H),7.20(dd,J= 9.1,2.7Hz,1H),6.59(d,J=5.5Hz,1H),6.31(d,J=6.7Hz,1H),6.26(d,J=6.0Hz,1H),6.00(d,J =12.0Hz,1H),5.80(d,J=12.0Hz,1H),4.37-4.35(m,1H),4.32-
4.30(m,1H),4.02(s,3H),3.92-3.86(m,2H),2.67-2.64(m,3H),0.83-0.77(m,2H),0.60-0.55(m,2H).

### Embodiment 2

### 4-[3-chloro-4-(cyclopropylaminocarbonyl)aminophenoxy]-6-[(1-diethylphosphorylacetyl-1,2,3,6-tetrahydropyridine)-4-yl]-7-methoxyquinoline (I-2)

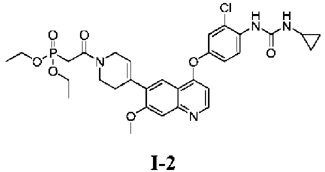

The preparation method refers to Embodiment 1, 4-bromo-3-methoxyaniline is used as a starting material, to obtain 30 mg of a yellow solid, yield: 47%, MS m/z: 644.2[M+H]⁺.
¹H-NMR(400MHz,DMSO-
d₆)δ(ppm):8.62(d,J=5.8Hz,1H),8.55(d,J=9.1Hz,1H),8.21(s,1H),8.07-
8.04(m,1H),7.84(s,1H),7.30(s,1H),7.16(d,J=8.6Hz,1H),6.71(d,J=5.5Hz,1H),6.03(d,J= 21.6Hz,1H),5.35-5.30(br.s,1H),4.34(d,J=11.8Hz,2H),4.24-
4.19(m,4H),4.11(s,3H),3.88(t,J=5.2Hz,1H),3.81(t,J=5.0Hz,1H),3.17(dd,J=22.0,11.0H z,2H),2.73-2.68(m,2H),2.62-2.57(m,1H),1.37(t,J=3.5Hz,6H),0.98-0.94(m,2H),0.82- 0.78(m,2H).

### Embodiment 3

### 4-[3-chloro-4-(cyclopropylaminocarbonyl)aminophenoxy]-6-[(1-ethylenesulfonyl- 1,2,3,6-tetrahydropyridine)-4-yl]-7-methoxyquinoline (I-3)

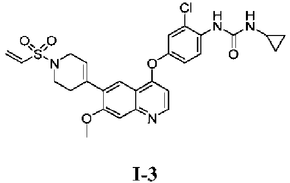

The preparation method refers to Embodiment 1, 4-bromo-3-methoxyaniline is used as a starting material, to obtain 20 mg of a yellow solid, yield: 36%, MS m/z: 556.2 [M+H]⁺.
¹H-NMR(400MHz,DMSO-
d₆)δ(ppm):8.55(d,J=5.4Hz,1H),8.19(d,J=9.0Hz,1H),8.10(s,1H),7.38(s,1H),7.37(d,J=2 .7Hz,1H),7.19(dd,J=9.0,2.7Hz,1H),6.74(dd,J=16.6,10.0Hz,1H),6.58(d,J=5.4Hz,1H),6 .26(d,J=16.5Hz,1H),6.14(d,J=10.1Hz,1H),4.01(s,3H),3.92-
3.88(m,2H),3.47(t,J=5.7Hz,2H),2.71-2.66(m,2H),2.64- 2.62(m,1H),2.64(dd,J=7.0,3.6Hz,1H),0.83-0.76(m,2H),0.61-0.53(m,2H).

### Embodiment 4

### 4-[3-chloro-4-(cyclopropylaminocarbonyl)aminophenoxy]-6-[(1-chloroyl-1,2,3,6- tetrahydropyridine)-4-yl]-7-methoxyquinoline (I-4)

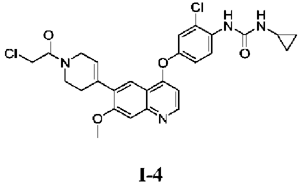

The preparation method refers to Embodiment 1, 4-bromo-3-methoxyaniline is used as a starting material, to obtain 20 mg of a yellow solid, yield: 37%, MS m/z: 542.3 [M+H]⁺.
¹H-NMR(400MHz,DMSO-
d₆)δ(ppm):8.64(br.s,1H),8.57(d,J=8.8Hz,1H),8.24(s,1H),8.14(s,1H),7.85(br.s,1H),7.3 1(s,1H),7.17(d,J=7.1Hz,1H),6.76(br.s,1H),6.04(t,J=17.8Hz,1H),4.31(d,J=10.7Hz,2H) ,4.19(s,2H),4.14(s,3H),3.89(m,1H),3.78(m,1H),2.71(m,2H),2.63(m,1H),0.97(m,2H),0 .81(m,2H).

### Embodiment 5

### 4-[3-chloro-4-cyclopropanecarboxylaminophenoxy]-6-[(1-chloroyl-1,2,3,6- tetrahydropyridine)-4-yl]-7-methoxyquinoline (I-5)

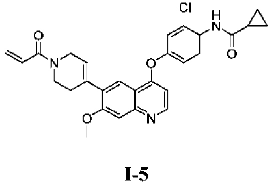

The preparation method refers to Embodiment 1, 4-bromo-3-methoxyaniline is used as a starting material, to obtain 50 mg of a yellow solid, yield: 50%, MS m/z: 504.3 [M+H]⁺.
¹H-NMR(400MHz,DMSO-
d₆)δ(ppm):8.64(br.s,1H),8.57(d,J=8.8Hz,1H),8.24(s,1H),8.14(s,1H),7.85(br.s,1H),7.3 1(s,1H),7.17(d,J=7.1Hz,1H),6.76(s,1H),6.04(d,J=17.8Hz,1H),4.35- 4.28(m,2H),4.19(s,2H),4.14(s,3H),3.92-3.87(m,1H),3.80-3.75(m,1H),2.78- 2.72(m,2H),2.65-2.60(m,1H),0.98-0.94(m,2H),0.87-0.84(m,2H).

### Embodiment 6

### 4-[3-chloro-4-cyclopentanylaminophenoxy]-6-[(1-chloroyl-1,2,3,6- tetrahydropyridine)-4-yl]-7-methoxyquinoline (I-6)

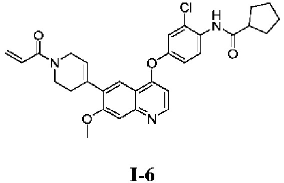

The preparation method refers to Embodiment 1, 4-bromo-3-methoxyaniline is used as a starting material to obtain 42 mg of a yellow solid, yield: 39%, MS m/z: 533.1 [M+H]⁺.
¹H-NMR(400MHz,DMSO-
d₆)δ(ppm):8.68(br.s,2H),8.22(s,1H),8.15(s,1H),7.76(s,1H),7.40- 7.30(m,1H),7.21(br.s,1H),6.74(br.s,1H),6.70-6.61(m,1H),6.38(d,J=16.7Hz,1H),6.08- 6.02(m,1H),5.77(d,J=10.3Hz,1H),4.38-4.32(m,2H),4.13(s,3H),3.92-3.82(m,2H),2.90- 2.82(m,1H),2.67-2.60(m,2H),2.08-2.01(m,2H),1.99-1.93(m,2H),1.87- 1.81(m,2H),1.73-1.69(m,2H).

### Embodiment 7

### 4-[3-chloro-4-cyclohexanecarbonylaminophenoxy]-6-[(1-chloroyl-1,2,3,6- tetrahydropyridine)-4-yl]-7-methoxyquinoline (I-7)

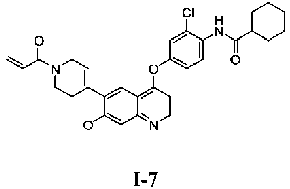

The preparation method refers to Embodiment 1, 4-bromo-3-methoxyaniline is used as a starting material, to obtain 45 mg of a yellow solid, yield: 41%, MS m/z: 547.1 [M+H]⁺.
¹H-NMR(400MHz,DMSO-
d₆)δ(ppm):8.68(d,J=8.4Hz,1H),8.22(s,1H),8.15(br.s,1H),7.79(s,1H),7.33(s,1H),7.19( d,J=8.2Hz,1H),6.75(br.s,1H),6.65(dd,J=16.9,10.3Hz,1H),6.38(br.s,1H),6.05(br.s,1H), 5.78(dd,J=10.5,1.8Hz,1H),4.37-4.32(m,2H),4.13(s,3H),3.90-3.82(m,2H),2.67- 2.62(m,2H),2.43-2.35(m,1H),2.10-2.03(m,2H),1.94-1.86(m,2H),1.79- 1.74(m,3H),1.65-1.59(m,3H).

### Embodiment 8

### 4-[3-chloro-4-(cyclopropylaminocarbonyl)aminophenoxy]-6-[(1-acryloyl- piperidine)-4-yl]-7-methoxyquinoline (I-8)

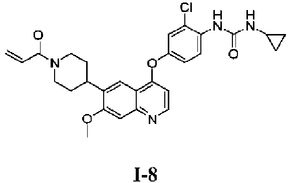

The preparation method refers to Embodiment 1, 4-bromo-3-methoxyaniline is used as a starting material, to obtain 20 mg of a yellow solid, yield: 38%, MS m/z: 522.2 [M+H]⁺.
¹H-NMR(400MHz,DMSO-d₆)δ(ppm):8.62(d,J=5.2Hz,1H),8.42(d,J=9.0Hz,1H), 8.09(s,1H),7.75(s,1H),7.53(s,1H),7.26(d,J=2.6Hz,1H),7.14(dd,J=9.0,2.5Hz,1H),6.50( d,J=5.0Hz,1H),6.41-6.33(m,1H),5.98- 5.89(m,1H),5.76(dd,J=10.6,1.6Hz,1H),5.59(s,1H),4.32(d,J=26.5Hz,2H),4.01(s,3H),3. 92(d,J=6.6Hz,1H),2.68(d,J=3.6Hz,3H),1.41- 1.29(m,3H),0.97(t,J=5.7Hz,2H),0.90(t,J=6.7Hz,1H),0.79(d,J=7.9Hz,2H).

### Embodiment 9

4-[3-chloro-4-(cyclopentylaminocarbonyl)aminophenoxy]-6-[(1-acryloyl-1,2,3,6- tetrahydropyridine)-4-yl]-7-methoxyquinoline (I-9)

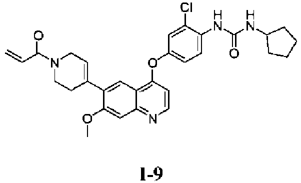

The preparation method refers to Embodiment 1, 4-bromo-3-methoxyaniline is used as a starting material, to obtain 25 mg of a yellow solid, yield: 46%, MS m/z: 548.2 [M+H]⁺.
¹H-NMR(400MHz,DMSO-
d₆)δ(ppm):8.57(br.s,1H),8.49(s,1H),8.23(br.s,1H),8.08(br.s,1H),7.60(br.s,1H),7.22(s, 1H),7.12(s,1H),6.72(br.s,1H),6.70-
6.61(m,1H),6.38(d,J=16.8Hz,1H),6.04(br.s,1H),5.78(d,J=10.2Hz,1H),4.40- 4.30(m,2H),4.12(s,3H),3.90-3.80(m,2H),3.43(t,J=7.0Hz,2H),2.67-2.60(m,2H),2.48- 2.42(m,2H),1.80-1.70(m,2H),1.68-1.62(m,2H),1.60-1.52(m,2H).

### Embodiment 10

### 4-[3-chloro-4-(cyclopropylaminocarbonyl)aminophenoxy]-6-[(1-acryloyl-1,2,3,6- tetrahydropyridine)-4-yl]-7-benzylquinoline (I-10)

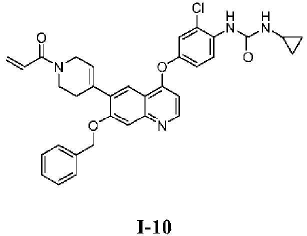

The preparation method refers to Embodiment 1, 4-bromo-3-methoxyaniline is used as a starting material, to obtain 20 mg of a yellow solid, yield: 34%, MS m/z: 596.2 [M+H]⁺.
¹H-NMR(400MHz,DMSO-
d₆)δ(ppm):8.59(d,J=5.3Hz,1H),8.27(d,J=9.1Hz,1H),8.02(s,1H),7.97(s,1H),7.51(d,J=8 .9Hz,2H),7.48-7.40(m,2H),7.35(t,J=7.2Hz,1H),7.29-
7.16(m,1H),6.83(ddd,J=27.3,16.6,10.3Hz,1H),6.51(d,J=5.2Hz,1H),6.22- 6.08(m,1H),5.98(d,J=13.7Hz,1H),5.76-
5.67(m,1H),5.35(s,2H),4.24(d,J=36.9Hz,2H),3.76(dd,J=23.1,17.0Hz,2H),2.71- 2.52(m,3H),0.76-0.57(m,2H),0.54-0.35(m,2H).

### Embodiment 11

### 4-[3-chloro-4-(cyclopropylaminocarbonyl)aminophenoxy]-6-[(1-acryloyl-1,2,3,6- tetrahydropyridine)-4-yl]-7-isopropylquinoline (I-11)

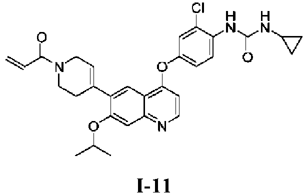

The preparation method refers to Embodiment 1, 4-bromo-3-methoxyaniline is used as a starting material, to obtain 30 mg of a yellow solid, yield: 55%, MS m/z: 548.2 [M+H]⁺.
¹H-NMR(400MHz,DMSO-
d₆)δ(ppm):8.57(d,J=4.8Hz,1H),8.44(d,J=9.0Hz,1H),8.12(d,J=15.0Hz,1H),7.78(s,1H), 7.67-
7.49(m,1H),7.24(d,J=2.3Hz,1H),7.09(t,J=20.1Hz,1H),6.66(td,J=17.0,10.7Hz,1H),6.5 1(d,J=4.3Hz,1H),6.44-
6.28(m,1H),5.96(d,J=27.4Hz,1H),5.75(d,J=10.7Hz,1H),5.62(s,1H),4.84(dt,J=11.7,5.8 Hz,1H),4.43-
4.16(m,2H),3.84(d,J=47.8Hz,2H),2.67(dd,J=6.3,3.2Hz,3H),1.46(d,J=5.9Hz,5H),0.95- 0.88(m,4H),0.78-0.75(m,2H).

### Embodiment 12

### 4-[3-chloro-4-(cyclopropylaminocarbonyl)aminophenoxy]-6-[(1-acryloyl-1,2,3,6- tetrahydropyridine)-4-yl]-7-cyclopentylquinoline (I-12)

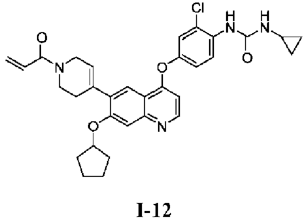

The preparation method refers to Embodiment 1, 4-bromo-3-methoxyaniline is used as a starting material, to obtain 15 mg of a yellow solid, yield: 26%, MS m/z: 574.2 [M+H]⁺.
¹H-NMR(400MHz,DMSO-
d₆)δ(ppm):8.55(d,J=4.9Hz,1H),8.35(t,J=25.3Hz,1H),8.05(s,1H),7.77(s,1H),7.38(s,1H ),7.20(s,1H),7.09(d,J=8.9Hz,1H),6.64(td,J=17.1,10.7Hz,1H),6.43(d,J=5.1Hz,1H),6.4 0-
6.25(m,1H),5.93(d,J=21.7Hz,1H),5.90(s,1H),5.73(d,J=10.6Hz,1H),5.17(s,1H),4.96(s, 1H),4.29(d,J=24.1Hz,2H),3.76(t,J=46.6Hz,2H),2.71-2.59(m,3H),2.15- 1.80(m,4H),1.85-1.58(m,4H),0.90-0.81(m,3H),0.71-0.62(m,3H).

### Embodiment 13

### 4-[3-chloro-4-(cyclopropylaminocarbonyl)aminophenoxy]-6-[(1-acryloyl-1,2,3,6- tetrahydropyridine)-4-yl]-7-ethylquinoline (I-13)

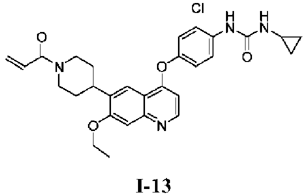

The preparation method refers to Emodiment 1, 4-bromo-3-methoxyaniline is used as a starting material, to obtain 10 mg of a yellow solid, yield: 19%, MS m/z: 534.4 [M+H]⁺.
¹H-NMR(400MHz,DMSO-
d₆)δ(ppm):8.59(d,J=5.2Hz,1H),8.42(d,J=9.0Hz,1H),8.08(s,1H),7.72(s,1H),7.39(d,J=1 1.6Hz,1H),7.25(dd,J=17.1,14.5Hz,2H),7.20(s,1H),6.66(td,J=17.2,10.6Hz,1H),6.46(d, J=5.2Hz,1H),6.37(dd,J=16.1,10.9Hz,1H),5.98(d,J=31.4Hz,1H),5.75(d,J=10.5Hz,1H), 5.25(s,1H),4.35(s,1H),4.32-
4.15(m,3H),3.91(t,J=5.4Hz,1H),3.79(t,J=5.4Hz,1H),2.68(dd,J=6.3,2.8Hz,3H),1.86(s, 2H),1.53(q,J=7.0Hz,3H),0.98-0.91(m,2H),0.91-0.84(m,2H).

### Embodiment 14

### 4-[3-chloro-4-(cyclopropylaminocarbonyl)aminophenoxy]-6-[(1-acryloyl-1,2,3,6- tetrahydropyridine)-4-yl]-7-(2-morpholinyl)ethylquinoline (I-14)

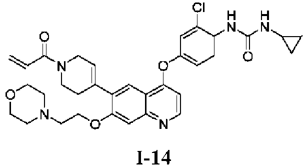

The preparation method refers to Embodiment 1, 4-bromo-3-methoxyaniline is used as a starting material, to obtain 18 mg of a yellow solid, yield: 29%, MS m/z: 619.2 [M+H]⁺.
¹H-NMR(400MHz,DMSO-
d₆)δ(ppm):8.64(d,J=6.2Hz,1H),8.53(d,J=9.2Hz,1H),8.21(s,1H),8.03(d,J=16.3Hz,1H), 7.83(s,1H),7.28(s,3H),7.15(d,J=9.1Hz,1H),6.68(d,J=16.6Hz,2H),6.39(d,J=16.4Hz,1H ),6.03(d,J=17.7Hz,1H),5.79(d,J=11.8Hz,1H),5.35(s,1H),4.70(s,2H),4.33(d,J=25.8Hz, 2H),4.00-3.90(m,3H),3.80-3.87(m,3H),3.38-3.25(m,2H),3.11-3.02(m,4H),1.02- 0.92(m,2H),0.85-0.79(m,2H).

### Embodiment 15

### 4-[3-chloro-4-(cyclohexylaminocarbonyl)aminophenoxy]-6-[(1-acryloyl-1,2,3,6- tetrahydropyridine)-4-yl]-7-methoxyquinoline (I-15)

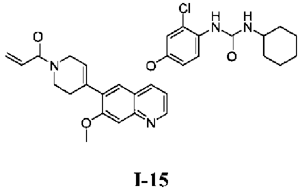

The preparation method refers to Embodiment 1, 4-bromo-3-methoxyaniline is used as a starting material, to obtain 25 mg of a yellow solid, yield: 44%, MS m/z: 562.2 [M+H]⁺.
¹H-NMR(400MHz,DMSO-
d₆)δ(ppm):8.59(d,J=5.3Hz,1H),8.31(d,J=9.0Hz,1H),8.07(s,1H),7.43(s,1H),7.22(d,J=2 .7Hz,1H),7.11(dd,J=9.0,2.7Hz,1H),6.85(d,J=9.3Hz,1H),6.73-
6.60(m,1H),6.47(d,J=4.8Hz,1H),6.37(dd,J=16.8,7.0Hz,1H),5.97(d,J=28.1Hz,1H),5.7 6(dd,J=10.6,1.9Hz,1H),5.01(dd,J=21.8,7.5Hz,1H),4.32(d,J=26.4Hz,2H),4.00(s,3H),3 .92(t,J=5.4Hz,1H),3.80(t,J=5.4Hz,1H),3.73-3.64(m,1H),2.70-2.62(m,2H),2.10- 2.01(m,2H), 1.83-1.74(m,3H), 1.47-1.35(m,2H), 1.25-1.15(m,3H).

### Embodiment 16

### 4-[3-chloro-4-(tert-butylaminocarbonyl)aminophenoxy]-6-[(1-acryloyl-1,2,3,6- tetrahydropyridine)-4-yl]-7-methoxyquinoline (I-16)

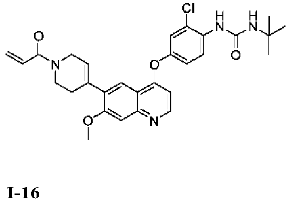

The preparation method refers to Embodiment 1, 4-bromo-3-methoxyaniline is used as a starting material, to obtain 40 mg of a yellow solid, yield: 37%, MS m/z: 536.2 [M+H]⁺.
¹H-NMR(400MHz,DMSO-
d₆)δ(ppm):8.58(d,J=5.3Hz,1H),8.29(d,J=9.0Hz,1H),8.07(s,1H),7.43(s,1H),7.21(d,J=2 .7Hz,1H),7.09(dd,J=9.0,2.6Hz,1H),6.83(d,J=12.6Hz,1H),6.73-
6.60(m,1H),6.46(d,J=4.7Hz,1H),6.37(dd,J=16.7,7.1Hz,1H),5.97(d,J=27.6Hz,1H),5.7 6(dd,J=10.6,1.7Hz,1H),5.10(d,J=25.1Hz,1H),4.37-4.34(m,1H),4.30-
4.27(m,1H),3.99(s,3H),3.92(t,J=5.3Hz,1H),3.80(t,J=5.3Hz,1H),2.70- 2.62(m,2H),1.45(s,9H).

### Embodiment 17

### 4-[3-chloro-4-(phenylaminocarbonyl)aminophenoxy]-6-[(1-acryloyl-1,2,3,6- tetrahydropyridine)-4-yl]-7-methoxyquinoline (I-17)

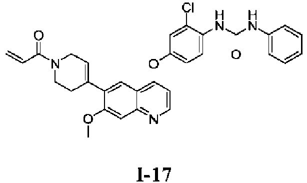

The preparation method refers to Embodiment 1, 4-bromo-3-methoxyaniline is used as a starting material, to obtain 57 mg of a yellow solid, yield: 51%, MS m/z: 556.2 [M+H]⁺.
¹H-NMR(400MHz,DMSO-d₆)δ(ppm):8.60-8.54(m,1H),8.41-
8.55(m,1H),8.07(d,J=8.8Hz,1H),7.75(d,J=26.2Hz,1H),7.52-
7.47(m,2H),7.43(s,1H),7.35(t,J=7.6Hz,2H),7.28(s,1H),7.21(d,J=2.6Hz,1H),7.12- 7.08(m,2H),6.77-6.62(m,1H),6.48-6.36(m,2H),5.99-
5.95(m,1H),5.80(d,J=10.5Hz,1H),4.42-4.36(m,1H),4.32-4.28(m,1H),3.99- 3.92(m,4H),3.84(t,J=5.5Hz,1H),2.71-2.62(m,2H).

### Embodiment 18

### 4-[2-fluoro-4-(cyclopropylaminocarbonyl)aminophenoxy]-6-[(1-acryloyl-1,2,3,6- tetrahydropyridine)-4-yl]-7-methoxyquinoline (I-18)

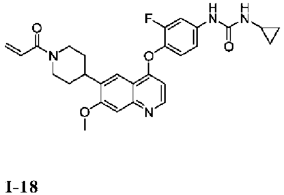

The preparation method refers to Embodiment 1, 4-bromo-3-methoxyaniline is used as a starting material, to obtain 32 mg of a yellow solid, yield: 64%, MS m/z: 503.2 [M+H]⁺.
¹H-NMR(400MHz,DMSO-
d₆)δ(ppm):8.80(s,1H),8.58(d,J=5.2Hz,1H),8.05(s,1H),7.77(s,2H),7.45(s,1H),6.85(dd d,J=36.4,16.8,10.5Hz,1H),6.73(s,1H),6.35(d,J=5.2Hz,1H),6.16(d,J=16.6Hz,1H),5.98 (d,J=19.5Hz,1H),5.73(t,J=8.3Hz,1H),4.25(d,J=37.8Hz,2H),3.96(s,3H),3.77(d,J=8.3H z,2H),2.60-2.56(m,3H),2.03-1.97(m,1H),0.72-0.58(m,2H),0.52-0.36(m,2H).

### Embodiment 19

### 4-[3-fluoro-4-(cyclopropylaminocarbonyl)aminophenoxy]-6-[(1-acryloyl-1,2,3,6- tetrahydropyridine)-4-yl]-7-methoxyquinoline (I-19)

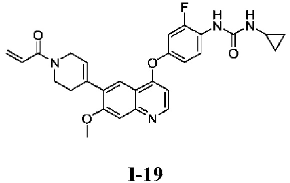

The preparation method refers to Embodiment 1, 4-bromo-3-methoxyaniline is used as a starting material, to obtain 35 mg of a yellow solid, yield: 70%, MS m/z: 503.2 [M+H]⁺.
¹H-NMR(400MHz,DMSO-d₆)δ(ppm):8.59(d,J=5.2Hz,1H),8.29-
8.13(m,2H),7.99(s,1H),7.42(s,1H),7.29(dd,J=11.8,2.5Hz,1H),7.06(d,J=9.0Hz,1H),6.9 5-6.73(m,2H),6.50(d,J=5.2Hz,1H),6.16(d,J=16.6Hz,1H),5.96(d,J=15.6Hz,1H),5.79- 5.66(m,1H),4.23(d,J=38.7Hz,2H),3.95(s,3H),3.82-3.66(m,2H),2.58-2.51(m,2H),2.01- 1.99(m,1H),0.66-0.60(m,2H),0.45-0.34(m,2H).

### Embodiment 20

### 4-[4-(cyclopropylaminocarbonyl)aminophenoxy]-6-[(1-acryloyl-1,2,3,6- tetrahydropyridine)-4-yl]-7-methoxyquinoline (I-20)

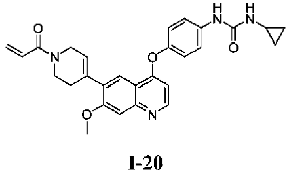

The preparation method refers to Embodiment 1, 4-bromo-3-methoxyaniline is used as a starting material, to obtain 25 mg of a yellow solid, yield: 52%, MS m/z: 485.2 [M+H]⁺.
¹H-NMR(400MHz,DMSO-d₆)δ(ppm):8.62-
8.39(m,2H),8.01(s,1H),7.53(t,J=8.5Hz,2H),7.41(s,1H),7.19- 7.08(m,2H),6.85(ddd,J=36.0,16.6,10.5Hz,1H),6.42(dd,J=10.9,3.7Hz,2H),6.16(d,J=1 6.3Hz,1H),5.97(d,J=15.9Hz,1H),5.77-
5.67(m,1H),4.23(d,J=38.2Hz,2H),3.95(s,3H),3.82-3.70(m,2H),2.63-2.54(m,2H),2.03- 1.97(m,1H),0.65-0.55(m,2H),0.47-0.36(m,2H).

### Embodiment 21

### 4-[3-chloro-4-(cyclopropylaminocarbonyl)aminophenoxy]-6-[(1-acryloyl-1,2,5,6- tetrahydropyridine)-3-yl]-7-methoxyquinoline (I-21)

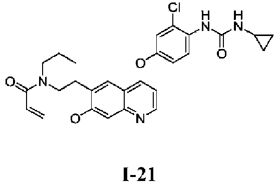

The preparation method refers to Embodiment 1, 4-bromo-3-methoxyaniline is used as a starting material, to obtain 13 mg of a yellow solid, yield: 25%, MS m/z: 519.2 [M+H]⁺.
¹H-NMR(400MHz,DMSO-
d₆)δ(ppm):8.61(s,1H),8.22(s,2H),7.89(s,1H),7.35(s,1H),7.18(s,1H),6.89(d,J=19.0Hz, 2H),6.50(s,2H),5.82(d,J=5.1Hz,1H),4.90(s,2H),4.67-
4.60(m,2H),4.22(s,3H),2.71(s,1H),2.50-2.43(m,2H),1.10-0.96(m,2H),0.90- 0.81(m,2H).

### Embodiment 22

### 4-[3-chloro-4-(cyclopropylaminocarbonyl)aminophenoxy]-6-[(1-acryloyl-2,5- dihydropyrrole)-3-yl]-7-methoxyquinoline (I-22)

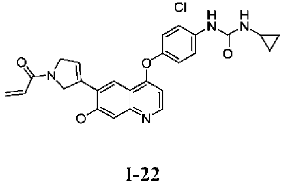

The preparation method refers to Embodiment 1, 4-bromo-3-methoxyaniline is used as a starting material, to obtain 10 mg of a yellow solid, yield: 20%, MS m/z: 505.2 [M+H]⁺.
¹H-NMR(400MHz,DMSO-
d₆)δ(ppm):8.59(s,1H),8.23(s,2H),7.88(s,1H),7.32(s,1H),7.19(s,1H),6.88(d,J=20.0Hz, 2H),6.52(s,2H),5.82(d,J=4.9Hz,1H),4.90(s,2H),4.67(d,J=25.6Hz,2H),4.22(s,3H),2.71 (s,1H),0.96(d,J=4.7Hz,3H),0.81(s,2H).

### Embodiment 23

### 4-[3-chloro-4-(cyclopropylaminocarbonyl)aminophenoxy]-6-[(1-acryloyl- tetrahydropyrrole)-3-yl]-7-methoxyquinoline (I-23)

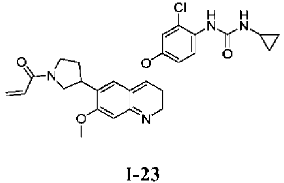

The preparation method refers to Embodiment 1, 4-bromo-3-methoxyaniline is used as a starting material, to obtain 19 mg of a yellow solid, yield: 38%, MS m/z: 507.2 [M+H]⁺.
¹H-NMR(400MHz,DMSO-
d₆)δ(ppm):8.64(d,J=5.1Hz,1H),8.41(d,J=9.0Hz,1H),8.10(s,1H),7.77(s,1H),7.54(s,1H) ,7.26(d,J=3.6Hz,1H),7.16(dd,J=9.0,2.5Hz,1H),6.50(d,J=5.1Hz,1H),6.41- 6.37(m,1H),5.98-
5.90(m,1H),5.76(dd,J=10.6,1.8Hz,1H),5.58(s,1H),4.32(d,J=20.5Hz,2H),4.00(s,3H),3. 92(d,J=6.6Hz,1H),2.68(d,J=3.6Hz,3H),1.41-1.30(m,1H),1.05- 0.97(m,2H),0.90(t,J=6.7Hz,1H),0.79(d,J=7.9Hz,2H).

### Embodiment 24

### 4-[3-chloro-4-(cyclopropylamino)carbonylphenoxy]-6-[(1-acryloyl-1,2,3,6- tetrahydropyridine)-4-yl]-7-methoxyquinoline (I-24)

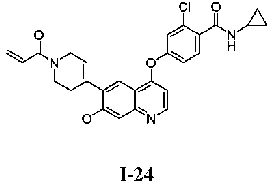

The preparation method refers to Embodiment 1, 4-bromo-3-methoxyaniline is used as a starting material, to obtain 12 mg of a yellow solid, yield: 24%, MS m/z: 505.2 [M+H]⁺.
¹H-NMR(400MHz,DMSO-
d₆)δ(ppm):8.66(br.s,1H),8.57(d,J=9.0Hz,1H),8.26(s,1H),8.15(s,1H),7.85(br.s,1H),7.3 3(s,1H),7.17(d,J=7.2Hz,1H),6.76(s,1H),6.06(d,J=18.0 Hz,1H),4.35- 4.29(m,2H),4.17(s,2H),4.16(s,3H),3.95-3.87(m,1H),3.82-3.75(m,1H),2.79- 2.72(m,2H),2.65-2.61(m,1H),0.98-0.90(m,2H),0.89-0.83(m,2H).

### Embodiment 25

### 4-[3-chloro-4-(cyclopropylaminocarbonyl)aminophenylmercapto]-6-[(1-acryloyl- 1,2,3,6-tetrahydropyridine)-4-yl]-7-methoxyquinoline (I-25)

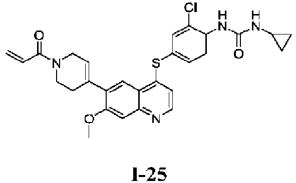

The preparation method refers to Embodiment 1, 4-bromo-3-methoxyaniline is used as a starting material, to obtain 9 mg of a yellow solid, yield: 17%, MS m/z: 536.2 [M+H]⁺.
₁H-NMR(400MHz,DMSO-
d₆)δ(ppm):8.55(d,J=5.5Hz,1H),8.21(d,J=9.0Hz,1H),8.13(s,1H),7.37(s,2H),7.19(dd,J= 9.0,2.8Hz,1H),6.58(d,J=5.6Hz,1H),6.30(d,J=6.7Hz,1H),6.24(d,J=6.0Hz,1H),6.01(d,J =12.0Hz,1H),5.82(d,J=12.0Hz,1H),4.37-4.34(m,1H),4.33-
4.30(m,1H),4.01(s,3H),3.93-3.86(m,2H),2.67-2.62(m,3H),0.83-0.78(m,2H),0.60- 0.53(m,2H).

### Embodiment 26

### 4-[3-chloro-4-(cyclopropylaminocarbonyl)aminophenylamino]-6-[(1-acryloyl- 1,2,3,6-tetrahydropyridine)-4-yl]-7-methoxyquinoline (I-26)

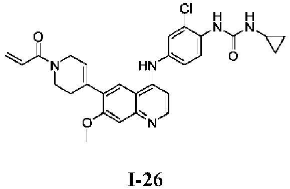

The preparation method refers to Embodiment 1, 4-bromo-3-methoxyaniline is used as a starting material, to obtain 23 mg of a yellow solid, yield: 44%, MS m/z: 519.2 [M+H]⁺.
¹H-NMR(400MHz,DMSO-
d₆)δ(ppm):8.62(d,J=5.6Hz,1H),8.25(d,J=9.0Hz,1H),8.17(s,1H),7.42(s,2H),7.21(dd,J= 9.2,2.7Hz,1H),6.60(d,J=5.6Hz,1H),6.35(d,J=6.8Hz,1H),6.28(d,J=6.0Hz,1H),6.02(d,J =12.0Hz,1H),5.85(d,J=12.0Hz,1H),4.38-4.35(m,1H),4.33- 4.30(m,1H),4.05(s,3H),3.92-3.84(m,2H),2.67-2.65(m,1H),2.65-2.62(m,2H),0.83- 0.77(m,2H),0.65-0.58(m,2H).

### Embodiment 27

### 4-[2,6-dichloro-4-(cyclopropylaminocarbonyl)aminobenzeneamino]-6-[(1- acryloyl-1,2,3,6-tetrahydropyridine)-4-yl]-7-methoxyquinoline (I-27)

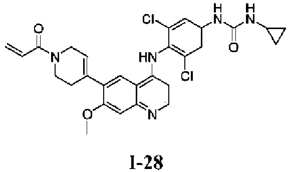

The preparation method refers to Embodiment 1, 4-bromo-3-methoxyaniline is used as a starting material, to obtain 11 mg of a yellow solid, yield: 20%, MS m/z: 553.2 [M+H]⁺.
¹H-NMR(400MHz,DMSO-
d₆)δ(ppm):8.70(s,1H),8.59(d,J=5.2Hz,1H),8.03(s,1H),7.73(d,J=13.6Hz,11H),7.43(s,1 H),7.33(t,J=9.0Hz,1 H),7.21(d,J=8.4Hz,1H),6.94-
6.75(m,2H),6.56(s,1H),6.43(d,J=5.1Hz,1H),6.16(d,J=17.0Hz,1H),5.98(d,J=15.5Hz,1 H),5.73(d,J=10.7Hz,1H),4.28(s,1H),4.19(s,1H),3.95(s,3H),3.76(s,2H),2.57- 2.51(m,1H),1.98-1.90(m,1H),0.67-0.62(m,2H),0.54-0.42(m,2H).

### Embodiment 28: Biological activity

### 1. Determination of FGFR4 inhibitory activity of target compound

The synthesized compound is determined by a fluorescence resonance energy transfer (FRET) method for the inhibitory activity of FGFR4 (reference of specific implementation methods: Lebakken CS,Kang HC,Vogel KW,A fluorescence lifetime- based binding assay to characterize kinase inhibitors.J Biomol Screen.2007.12(6):828-841.), and compared with a positive control drug, to screen out the compound with the better activity. FGFR4 is obtained by direct purchase of a kit.

### 2. Determination of Hep3B cell inhibitory activity of target compound

a. cells are recovered, and cultured to a logarithmic growth phase; b. compound preparation: a test sample is dissolved in dimethylsulfoxide (DMSO), the concentration is 30 mM, it is diluted in a gradient at a ratio of 1:3, and there is a total of 10 gradients; c. the cells at a density of 4~6*10⁴/ml are inoculated into a 384-well plate at 25 µL per well, the compound is added, and the final concentration of the compound is 30, 10, 3.33, 1.11, 0.37, 0.123, 0.041, 0.014, 0.005, and 0.002 mM; d. the compound is incubated with the cells for 72 h, and the cell activity is detected by a Cell Titer-Glo (CTG) method.

The following table shows in vitro FGFR4 kinase activity and in vitro cancer cell activity test results of some compounds:

| Compound code | In vitro FGFR4 kinase inhibitory activity IC₅₀ (nM) | Hep3B cell inhibitory activity IC₅₀ (nM) |
|---|---|---|
| I-1 | <2 | <50 |
| I-2 | <5 | <100 |
| I-3 | <5 | <100 |
| I-4 | <5 | <100 |
| I-5 | <5 | <100 |
| I-6 | <5 | <100 |
| I-7 | <2 | <50 |
| I-8 | <2 | <50 |
| I-9 | <5 | <100 |
| I-10 | <5 | <100 |
| I-11 | <2 | <50 |
| I-12 | <5 | <100 |
| I-13 | <2 | <50 |
| I-14 | <5 | <100 |
| I-15 | <2 | <50 |
| I-16 | <5 | <50 |
| I-17 | <2 | <50 |
| I-18 | <5 | <50 |
| I-19 | <2 | <50 |
| I-20 | <5 | <500 |
| I-21 | <5 | <500 |
| I-22 | <5 | <100 |
| I-23 | <5 | <500 |
| I-24 | <2 | <50 |
| I-25 | <5 | <100 |
| I-26 | <5 | <500 |
| I-27 | <5 | <500 |
| Blu9931 | 1.78 | 174 |
| Lenvatinib | 43 | <500 |

(The compound code in the table corresponds to the previous compound code)

It may be seen from the above table that: the above compound of the present disclosure and the medically acceptable salt thereof have the FGFR4 inhibitory effect, and may provide a basis for the preparation of the drug for treating/preventing the FGFR4-related diseases.

It is indicated from pharmacological test results that the quinoline compound in the present disclosure has the better FGFR4 kinase and Hep3B cell inhibitory activities, and IC₅₀ values of some compounds are comparable to or better than the positive control drugs Blu9931 and Lenvatinib, and may be used for preventing or treating clinical diseases associated with FGFR4 kinase inhibitors.

The above compound of the present disclosure and the medically acceptable salt thereof has the FGFR4 inhibitory effect, and may be used as an active ingredient in pharmaceuticals. Therefore, the drug containing the above compound as the active ingredient may be used to prepare a drug for treating clinical symptoms related to FGFR4, such as: the use in preparation of the drug for preventing and/or treating the diseases related to FGFR4-related abnormal cell proliferation, morphological changes and hyperkinesias and the like in the organisms and the drug for the diseases associated with angiogenesis or cancer metastasis.

## Claims

1. A compound of Formula I or a pharmaceutically acceptable salt, crystal form, stereoisomer, tautomer, hydrate or solvate thereof: 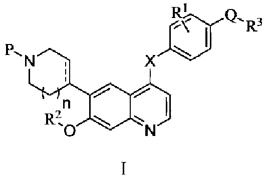 in Formula I,
X is selected from -CR⁴R⁵-, -NR⁴-, O or S, wherein R⁴ and R⁵ each independently represent hydrogen, deuterium, halogen, alkyl, aryl or Het;
Q is selected from -NR⁶CONR⁷-, -CONR⁶-, -NR⁶CO-, -NR⁶SO₂NR⁷-, -SO₂NR⁶-,-NR⁶SO₂-, -NR⁶-, -NR⁶(CH₂)mN-, -NR⁶(CH₂)mO- or -CR⁶R⁷, wherein m=1, 2, 3, 4 or 5, R⁶, and R⁷ each independently represent hydrogen, deuterium, alkyl, aryl or Het;
a dotted line represents that a single-band or a double-bond may be present; n=0, 1, 2 or 3;
R¹ is selected from hydrogen, halogen, hydroxyl, alkoxy, alkyl, aryl or Het;
R² is selected from hydrogen, halogen, hydroxyl, alkoxy, alkyl, aryl or Het;
R³ is selected from hydrogen, halogen, hydroxyl, alkoxy, alkyl, aryl or Het;
P is selected from the following structures: 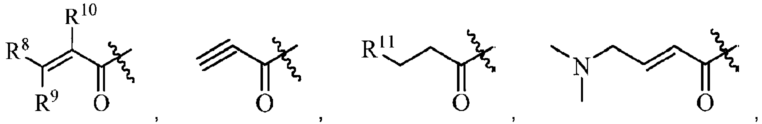 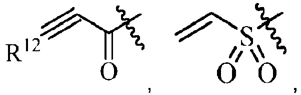
wherein, R¹¹ is a leaving group or an activated ester; and the leaving group is a halogen; R⁸, R⁹, R¹⁰, and R¹² are each independently selected from hydrogen, halogen, hydroxyl, alkoxy, alkyl, aryl or Het;
the alkyl is a straight-chain or branched-chain saturated hydrocarbyl of 1-6 carbon atoms, a cyclic saturated hydrocarbyl of 3-6 carbon atoms, or a cyclic saturated hydrocarbyl of 3-6 carbon atoms linked with a straight-chain or branched-chain saturated hydrocarbyl of 1-6 carbon atoms;
in the above groups, the aryl is a carbocyclic ring selected from phenyl, naphthyl, acenaphthyl or tetrahydronaphthyl, each of which is optionally substituted by 1, 2 or 3 substituents, and each substituent is independently selected from hydrogen, alkyl, cyano, halogen, nitro, haloalkyl, hydroxyl, mercapto, alkoxy, alkylthio, alkoxyalkyl, aralkyl, diarylalkyl, aryl or Het;
Het is a monocyclic heterocycle selected from piperidinyl, pyrrolyl, pyrazolyl, imidazolyl, furyl, thienyl, oxazolyl, isoxazolyl, thiazolyl, isothiazolyl, pyridyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxenyl or benzo[1,3]dioxolyl; and each monocyclic or bicyclic heterocycle is optionally substituted with 1, 2 or 3 substituents, and each substituent is independently selected from halogen, haloalkyl, hydroxy, alkyl or alkoxy; and
the halogen is selected from fluorine, chlorine, bromine or iodine.

2. The compound according to claim 1, wherein in Formula I,
X is -NR⁴- or O, wherein R⁴ is selected from hydrogen, deuterium, halogen, alkyl, aryl or Het;
Q is selected from -NR⁶CONR⁷-, -CONR⁶-, -NR⁶CO-, -NR⁶SO₂NR⁷-, -SO₂NR⁶- or -NR⁶SO₂-, wherein R⁶, and R⁷ each independently represent hydrogen, deuterium, alkyl, aryl or Het;
a dotted line indicates that a single-bond or a double-bond may be present;
n=0, 1, 2 or 3;
R¹ is selected from hydrogen, halogen or alkyl;
R² is selected from hydrogen, alkyl or aryl; and
R³ is selected from alkoxy, alkyl, aryl or Het.

3. The compound according to claim 1, wherein in Formula I,
X is O;
Q is selected from -NR⁶CONR⁷-, -CONR⁶- or -NR⁶CO-, wherein R⁶, and R⁷ each independently represent hydrogen, deuterium, alkyl, aryl or Het;
a dotted line indicates that a single-bond or a double-bond may be present;
n=0, 1, 2 or 3;
R¹ is selected from hydrogen, halogen or alkyl;
R² is alkyl or aryl; and
R³ is selected from alkyl, aryl or Het.

4. The compound according to claim 1, wherein in Formula I,
X is O;
Q is selected from -NR⁶CONR⁷-, -CONR⁶- or -NR⁶CO-, wherein R⁶, and R⁷ each independently represent hydrogen, deuterium, alkyl, aryl or Het;
a dotted line indicates that a single-bond or a double-bond may be present;
n=0 or 1;
R¹ is hydrogen or halogen;
R² is alkyl or aryl;
R³ is selected from alkyl, aryl or Het; and
P is selected from the following structures: 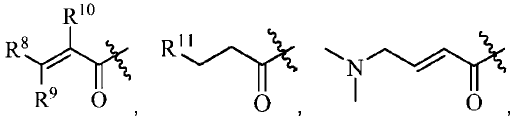
wherein, R¹¹ is a leaving group or an activated ester, and the leaving group is a halogen; R⁸, R⁹ and R¹⁰ are each independently selected from hydrogen or alkyl.

5. The compound according to claim 1, wherein the pharmaceutically acceptable salt comprises an acid addition salt formed by the compound of Formula I with the following acids: hydrochloric acid, hydrobromic acid, sulfuric acid, phosphoric acid, methanesulfonic acid, benzenesulfonic acid, p-toluenesulfonic acid, naphthalenesulfonic acid, citric acid, tartaric acid, lactic acid, pyruvic acid, acetic acid, maleic acid or succinic acid, fumaric acid, salicylic acid, phenylacetic acid, and mandelic acid; and also comprises an acidic salt formed by the compound of Formula I with an inorganic base.

6. The compound according to claim 5, wherein the pharmaceutically acceptable salt comprises an alkaline metal cation salt, an alkaline earth metal cation salt and an ammonium cation salt.

7. The compound according to claim 1, wherein the compound of Formula I is one of the following compounds:

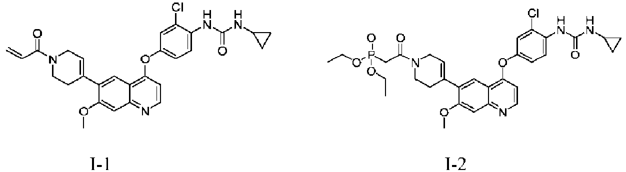

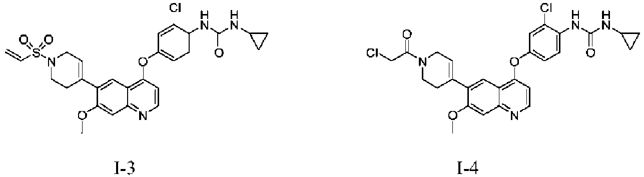

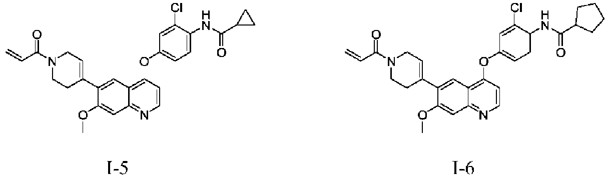

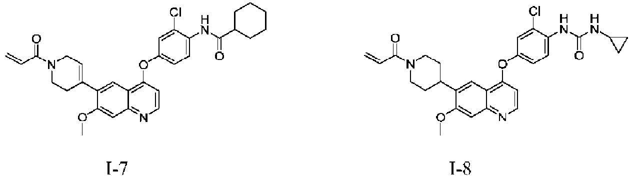

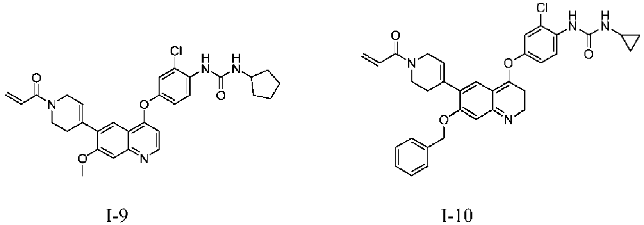

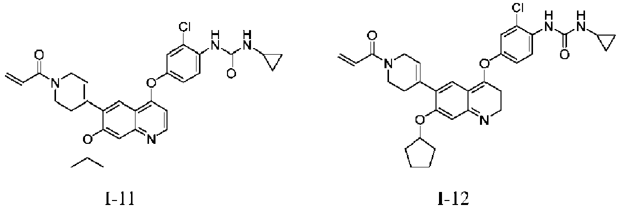

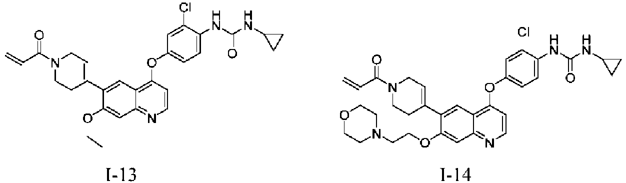

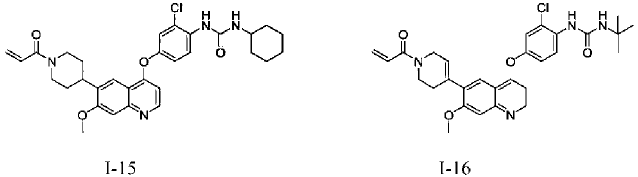

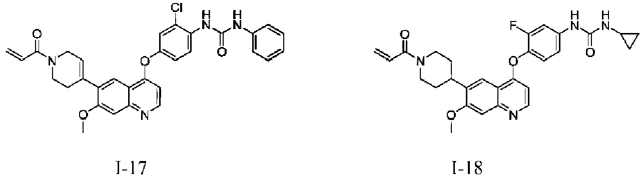

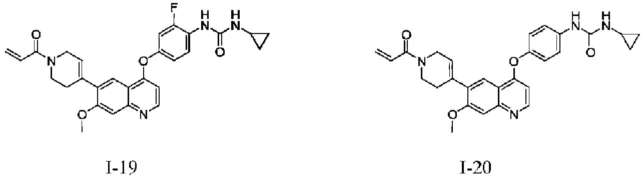

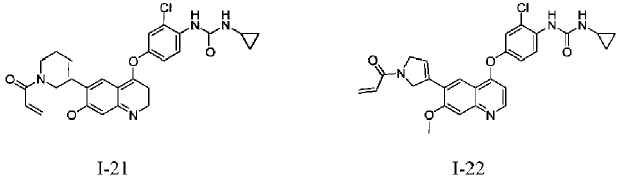

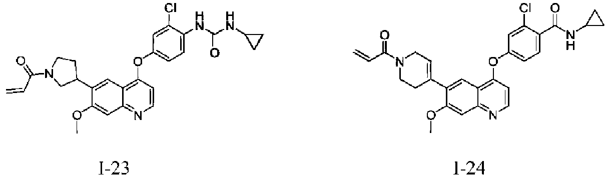

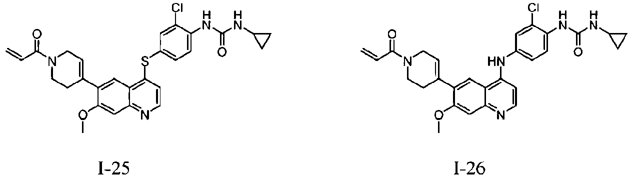

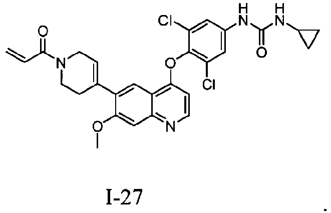

8. A pharmaceutical composition, comprising the compound or the pharmaceutically acceptable salt, crystal form, stereoisomer, tautomer, hydrate or solvate thereof according to claim 1, and a pharmaceutically acceptable carrier or excipient.

9. The compound according to claim 1 for use in prevention and/or treatment of FGFR4-related diseases; the FGFR4-related disease is selected from hyperlipidemia or cancer; and the cancer comprises lung cancer, squamous cell carcinoma, bladder cancer, gastric cancer, ovarian cancer, peritoneal cancer, breast cancer, breast ductal carcinoma, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin lymphoma, colorectal villous adenomas, melanomas, cell tumor or sarcoma, or myelodysplastic syndrome.

## Patentansprüche

1. Eine Verbindung der Formel I oder ein pharmazeutisch verträgliches Salz, eine Kristallform, ein Stereoisomer, ein Tautomer, ein Hydrat oder ein Solvat davon: 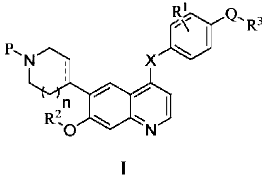 in Formel I
X ist ausgewählt aus -CR⁴R⁵-, -NR⁴-, O oder S, wobei R⁴ und R⁵ jeweils unabhängig voneinander Wasserstoff, Deuterium, Halogen, Alkyl, Aryl oder Het darstellen;
Q ist ausgewählt aus -NR⁶CONR⁷ -, -CONR⁶ -, -NR⁶ CO-, -NR⁶ SO₂ NR⁷ -, -SO₂ NR⁶ -, -NR⁶ SO₂ -, -NR⁶ -, -NR⁶ (CH₂ )mN-, -NR⁶ (CH₂ )mO- oder -CR⁶ R⁷, wobei m = 1, 2, 3, 4 oder 5, R⁶ und R⁷ jeweils unabhängig voneinander Wasserstoff, Deuterium, Alkyl, Aryl oder Het bedeuten;
eine gestrichelte Linie stellt dar, dass eine Einfachbindung oder eine Doppelbindung vorhanden sein kann;
n = 0, 1, 2 oder 3;
R¹ ist ausgewählt aus Wasserstoff, Halogen, Hydroxyl, Alkoxy, Alkyl, Aryl oder Het ausgewählt ist;
R² ist ausgewählt aus Wasserstoff, Halogen, Hydroxyl, Alkoxy, Alkyl, Aryl oder Het;
R³ ist ausgewählt aus Wasserstoff, Halogen, Hydroxyl, Alkoxy, Alkyl, Aryl oder Het;
P ist ausgewählt aus den folgenden Strukturen: 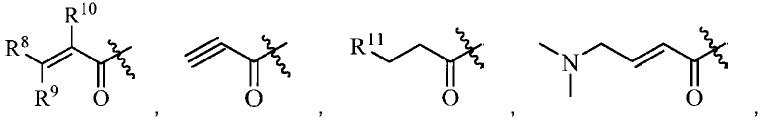 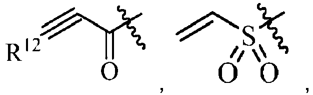
wobei R¹¹ eine Abgangsgruppe oder ein aktivierter Ester ist; und die Abgangsgruppe ein Halogen ist; R⁸, R⁹, R¹⁰ und R¹² jeweils unabhängig voneinander aus Wasserstoff, Halogen, Hydroxyl, Alkoxy, Alkyl, Aryl oder Het ausgewählt sind;
das Alkyl ist ein geradkettiger oder verzweigter gesättigter Kohlenwasserstoffrest mit 1 bis 6 Kohlenstoffatomen, ein cyclischer gesättigter Kohlenwasserstoffrest mit 3 bis 6 Kohlenstoffatomen oder ein cyclischer gesättigter Kohlenwasserstoffrest mit 3 bis 6 Kohlenstoffatomen ist, der mit einem geradkettigen oder verzweigten gesättigten Kohlenwasserstoffrest mit 1 bis 6 Kohlenstoffatomen verbunden ist;
in den oben genannten Gruppen ist das Aryl ein carbocyclischer Ring, ausgewählt aus Phenyl, Naphthyl, Acenaphthyl oder Tetrahydronaphthyl, von denen jeder gegebenenfalls durch 1, 2 oder 3 Substituenten substituiert ist, und jeder Substituent unabhängig ausgewählt ist aus Wasserstoff, Alkyl, Cyano, Halogen, Nitro, Haloalkyl, Hydroxyl, Mercapto, Alkoxy, Alkylthio, Alkoxyalkyl, Aralkyl, Diarylalkyl, Aryl oder Het;
Het ist ein monocyclischer Heterocyclus ist, ausgewählt aus Piperidinyl, Pyrrolyl, Pyrazolyl, Imidazolyl, Furyl, Thienyl, Oxazolyl, Isoxazolyl, Thiazolyl, Isothiazolyl, Pyridyl, Pyrimidinyl, Pyrazinyl oder Pyridazinyl; oder ein bicyclischer Heterocyclus, ausgewählt aus Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxenyl oder Benzo[1,3]dioxolyl; und jeder monocyclische oder bicyclische Heterocyclus ist gegebenenfalls mit 1, 2 oder 3 Substituenten substituiert, wobei jeder Substituent unabhängig voneinander aus Halogen, Haloalkyl, Hydroxy, Alkyl oder Alkoxy ausgewählt ist; und
wobei das Halogen aus Fluor, Chlor, Brom oder Jod ausgewählt ist.

2. Die Verbindung nach Anspruch 1, wobei in Formel I
X -NR⁴- oder O ist, wobei R⁴ aus Wasserstoff, Deuterium, Halogen, Alkyl, Aryl oder Het ausgewählt ist;
Q ausgewählt ist aus -NR⁶CONR⁷ -, -CONR⁶ -, -NR⁶ CO-, -NR⁶ SO₂ NR⁷ -, -SO₂ NR⁶ - oder -NR⁶ SO₂ -, wobei R⁶ und R⁷ jeweils unabhängig voneinander Wasserstoff, Deuterium, Alkyl, Aryl oder Het darstellen;
eine gestrichelte Linie darstellt, dass eine Einfachbindung oder eine Doppelbindung vorhanden sein kann;
n = 0, 1, 2 oder 3;
R¹ ist aus Wasserstoff, Halogen oder Alkyl ausgewählt ist;
R² ist ausgewählt aus Wasserstoff, Alkyl oder Aryl; und
R³ ist ausgewählt aus Alkoxy, Alkyl, Aryl oder Het.

3. Die Verbindung nach Anspruch 1, wobei in Formel I
X ist O;
Q ist aus -NR⁶CONR⁷ -, -CONR⁶ - oder -NR⁶ CO- ausgewählt ist, wobei R⁶ und R⁷ jeweils unabhängig voneinander Wasserstoff, Deuterium, Alkyl, Aryl oder Het darstellen;
eine gestrichelte Linie darstellt, dass eine Einfachbindung oder eine Doppelbindung vorhanden sein kann;
n = 0, 1, 2 oder 3;
R¹ ist ausgewählt aus Wasserstoff, Halogen oder Alkyl;
R² ist Alkyl oder Aryl; und
R³ ist ausgewählt aus Alkyl, Aryl oder Het.

4. Die Verbindung nach Anspruch 1, wobei in Formel I
X ist O;
Q ist aus -NR⁶CONR⁷ -, -CONR⁶ - oder -NR⁶ CO- ausgewählt ist, wobei R⁶ und R⁷ jeweils unabhängig voneinander Wasserstoff, Deuterium, Alkyl, Aryl oder Het darstellen;
eine gestrichelte Linie darstellt, dass eine Einfachbindung oder eine Doppelbindung vorhanden sein kann;
n = 0 oder 1;
R¹ Wasserstoff oder Halogen ist;
R² ist Alkyl oder Aryl;
R³ ist ausgewählt aus Alkyl, Aryl oder Het; und
P ist ausgewählt aus den folgenden Strukturen: 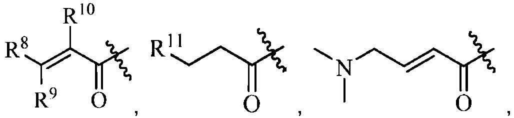
wobei R¹¹ eine Abgangsgruppe oder ein aktivierter Ester ist und die Abgangsgruppe ein Halogen ist; R⁸, R⁹ und R¹⁰ jeweils unabhängig voneinander aus Wasserstoff oder Alkyl ausgewählt sind.

5. Die Verbindung gemäß Anspruch 1, wobei das pharmazeutisch verträgliche Salz ein Säureadditionssalz umfasst, das durch die Verbindung der Formel I mit den folgenden Säuren gebildet wird: Salzsäure, Bromwasserstoffsäure, Schwefelsäure, Phosphorsäure, Methansulfonsäure, Benzolsulfonsäure, p-Toluolsulfonsäure, Naphthalinsulfonsäure, Zitronensäure, Weinsäure, Milchsäure, Brenztraubensäure, Essigsäure, Maleinsäure oder Bernsteinsäure, Fumarsäure, Salicylsäure, Phenylessigsäure und Mandelsäure; und ferner ein Säuresalz umfasst, das durch die Verbindung der Formel I mit einer anorganischen Base gebildet wird.

6. Die Verbindung gemäß Anspruch 5, wobei das pharmazeutisch verträgliche Salz ein Alkalimetallkationsalz, ein Erdalkalimetallkationsalz und ein Ammoniumkationsalz umfasst.

7. Die Verbindung gemäß Anspruch 1, wobei die Verbindung der Formel I eine der folgenden Verbindungen ist:

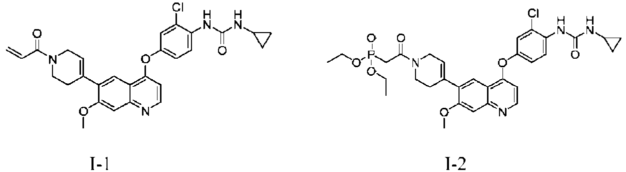

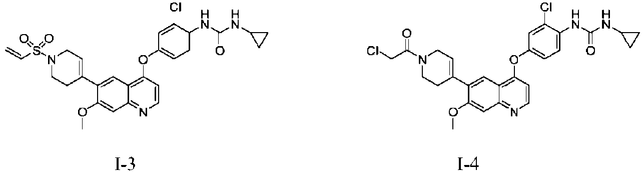

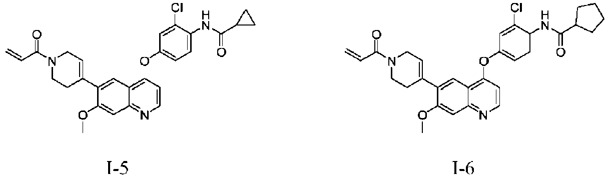

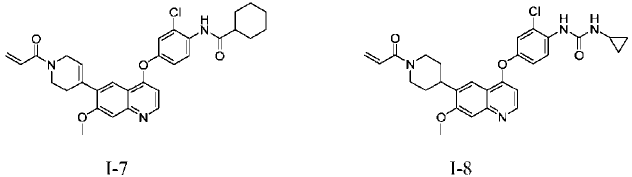

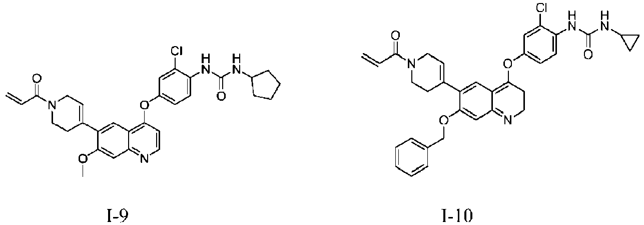

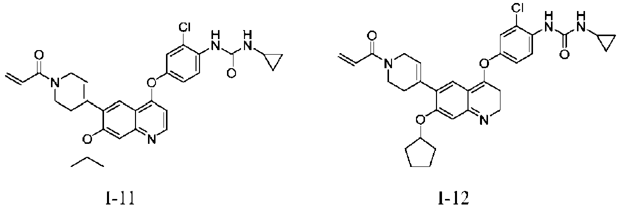

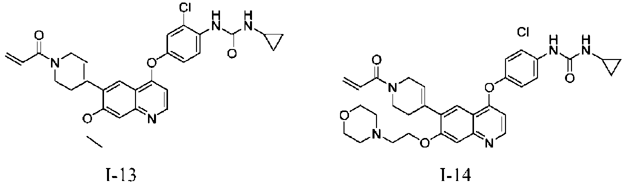

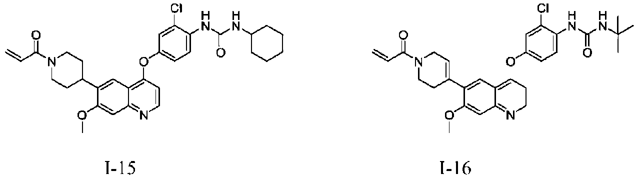

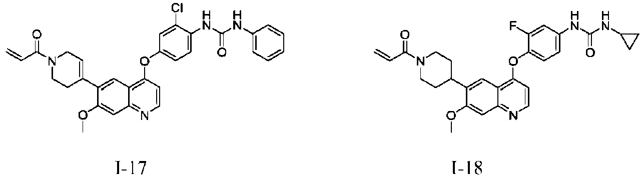

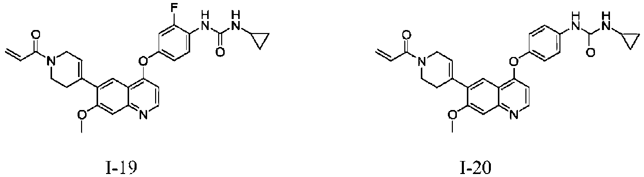

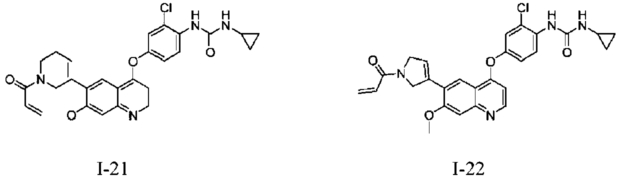

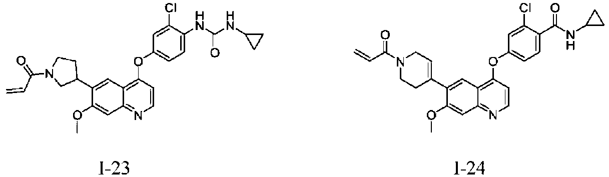

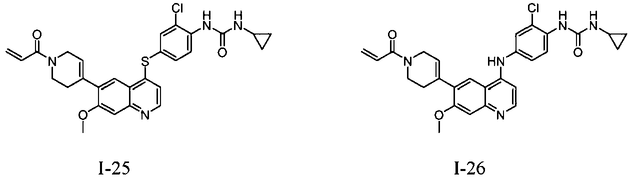

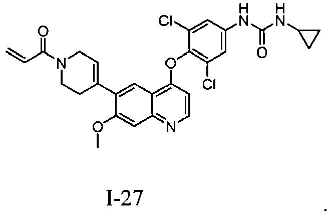

8. Eine pharmazeutische Zusammensetzung, umfassend die Verbindung oder das pharmazeutisch verträgliche Salz, die Kristallform, das Stereoisomer, das Tautomer, das Hydrat oder das Solvat davon gemäß Anspruch 1 sowie einen pharmazeutisch verträglichen Träger oder Hilfsstoff.

9. Die Verbindung gemäß Anspruch 1 zur Verwendung bei der Vorbeugung und/oder Behandlung von FGFR4-assoziierten Erkrankungen; wobei die FGFR4-assoziierte Erkrankung aus Hyperlipidämie oder Krebs ausgewählt ist; und wobei der Krebs Lungenkrebs, Plattenepithelkarzinom, Blasenkrebs, Magenkrebs, Eierstockkrebs, Peritonealkrebs, Brustkrebs, duktales Brustkarzinom, Kopf-Hals-Krebs, Endometriumkarzinom, Gebärmutterkrebs, Rektumkrebs, Leberkrebs, Nierenkrebs, Nierenbeckenkrebs, Speiseröhrenkrebs, Adenokarzinom der Speiseröhre, Gliom, Prostatakrebs, Schilddrüsenkrebs, Krebs des weiblichen Reproduktionssystems, Karzinom in situ, Lymphom, Neurofibromatose, Knochenkrebs, Hautkrebs, Hirnkrebs, Dickdarmkrebs, Hodenkrebs, gastrointestinaler Stromatumor, Mundhöhlenkrebs, Rachenkrebs, multiples Myelom, Leukämie, Non-Hodgkin-Lymphom, kolorektale villöse Adenome, Melanome, Zelltumor oder Sarkom oder myelodysplastisches Syndrom umfasst.

## Revendications

1. Composé de formule I ou un de ses sels, formes cristallines, stéréoisomères, tautomères, hydrates ou solvates pharmaceutiquement acceptables : 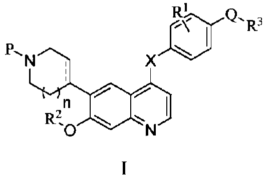 dans la formule I,
X est choisi parmi -CR⁴R⁵-, -NR⁴-, O ou S, où R⁴ et R⁵représentent chacun indépendamment un atome d'hydrogène, de deutérium, d'halogène, un groupe alkyle, aryle ou Het ;
Q est choisi parmi -NR⁶ CONR⁷ -, -CONR⁶ -, -NR⁶ CO-, -NR⁶ SO₂ NR⁷ -, -SO₂ NR⁶ -, -NR⁶ SO₂ -, -NR⁶ -, -NR⁶ (CH₂ )mN-, -NR⁶ (CH₂ )mO- ou -CR⁶ R⁷ , où m = 1, 2, 3, 4 ou 5, R⁶ et R⁷ représentent chacun indépendamment un atome d'hydrogène, de deutérium, un groupe alkyle, aryle ou Het ;
une ligne pointillée indique qu'une liaison simple ou une liaison double peut être présente ;
n = 0, 1, 2 ou 3 ;
R¹ est choisi parmi l'hydrogène, un halogène, un groupe hydroxyle, alcoxy, alkyle, aryle ou Het ;
R² est choisi parmi l'hydrogène, un halogène, un groupe hydroxyle, alcoxy, alkyle, aryle ou Het ;
R³ est choisi parmi l'hydrogène, un halogène, un groupe hydroxyle, alcoxy, alkyle, aryle ou Het ;
P est choisi parmi les structures suivantes : 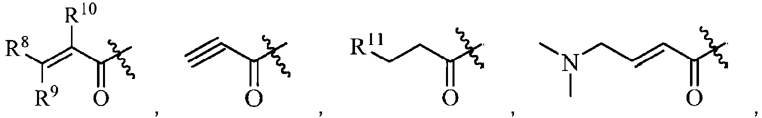 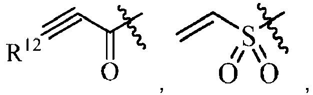
dans lesquelles R¹¹ est un groupe partant ou un ester activé ; et le groupe partant est un halogène ; R⁸ , R⁹ , R¹⁰ et R¹² sont chacun choisis indépendamment parmi l'hydrogène, un halogène, un groupe hydroxyle, alcoxy, alkyle, aryle ou Het ;
l'alkyle est un groupe hydrocarbyle saturé à chaîne linéaire ou ramifiée de 1 à 6 atomes de carbone, un groupe hydrocarbyle saturé cyclique de 3 à 6 atomes de carbone, ou un groupe hydrocarbyle saturé cyclique de 3 à 6 atomes de carbone lié à un groupe hydrocarbyle saturé à chaîne linéaire ou ramifiée de 1 à 6 atomes de carbone ;
dans les groupes ci-dessus, le groupe aryle est un cycle carbocyclique choisi parmi les groupes phényle, naphtyle, acénaphtyle ou tétrahydronaphtyle, chacun d'entre eux pouvant être éventuellement substitué par 1, 2 ou 3 substituants, et chaque substituant est choisi indépendamment parmi l'hydrogène, les groupes alkyle, cyano, halogéno, nitro, halogénoalkyle, hydroxyle, mercapto, alcoxy, alkylthio, alcoxyalkyle, aralkyle, diarylalkyle, aryle ou Het ;
Het est un hétérocycle monocyclique choisi parmi les groupes pipéridinyle, pyrrolyle, pyrazolyle, imidazolyle, furyle, thiényle, oxazolyle, isoxazolyle, thiazolyle, isothiazolyle, pyridyle, pyrimidinyle, pyrazinyle ou pyridazinyle ; ou un hétérocycle bicyclique choisi parmi les groupes quinolinyle, quinoxalinyle, indolyle, benzimidazolyle, benzoxazolyle, benzisoxazolyle, benzothiazolyle, benzisothiazolyle, benzofuranyle, benzothiényle, 2,3-dihydrobenzo[1,4]dioxényle ou benzo[1,3]dioxolyle ; et chaque hétérocycle monocyclique ou bicyclique est éventuellement substitué par 1, 2 ou 3 substituants, et chaque substituant est choisi indépendamment parmi un halogène, un halogénoalkyle, un groupe hydroxy, alkyle ou alcoxy ; et
l'halogène est choisi parmi le fluor, le chlore, le brome ou l'iode.

2. Composé selon la revendication 1, dans lequel, dans la formule I,
X représente -NR⁴ - ou O, où R⁴ est choisi parmi l'hydrogène, le deutérium, un halogène, un groupe alkyle, aryle ou Het ;
Q est choisi parmi -NR⁶ CONR⁷ -, -CONR⁶ -, -NR⁶ CO-, -NR⁶ SO₂ NR⁷ -, -SO₂ NR⁶ - ou -NR⁶ SO₂ -, où R⁶ et R⁷ représentent chacun indépendamment un atome d'hydrogène, de deutérium, un groupe alkyle, aryle ou Het ;
une ligne pointillée indique qu'une liaison simple ou une liaison double peut être présente ;
n = 0, 1, 2 ou 3 ;
R¹ est choisi parmi l'hydrogène, un halogène ou un groupe alkyle ;
R² est choisi parmi l'hydrogène, un groupe alkyle ou aryle ; et
R³ est choisi parmi un groupe alcoxy, alkyle, aryle ou Het.

3. Composé selon la revendication 1, dans lequel, dans la formule I,
X représente O ;
Q est choisi parmi -NR⁶ CONR⁷ -, -CONR⁶ - ou -NR⁶ CO-, où R⁶ et R⁷ représentent chacun indépendamment un atome d'hydrogène, de deutérium, un groupe alkyle, aryle ou Het ;
une ligne pointillée indique qu'il peut y avoir une liaison simple ou une liaison double ;
n = 0, 1, 2 ou 3 ;
R¹ est choisi parmi l'hydrogène, un halogène ou un groupe alkyle ;
R² est un groupe alkyle ou aryle ; et
R³ est choisi parmi un groupe alkyle, aryle ou Het.

4. Composé selon la revendication 1, dans lequel, dans la formule I,
X représente O ;
Q est choisi parmi -NR⁶ CONR⁷ -, -CONR⁶ - ou -NR⁶ CO-, où R⁶ et R⁷ représentent chacun indépendamment un atome d'hydrogène, de deutérium, un groupe alkyle, aryle ou Het ;
une ligne pointillée indique qu'une liaison simple ou une liaison double peut être présente ;
n = 0 ou 1 ;
R¹ est un atome d'hydrogène ou d'halogène ;
R² est un groupe alkyle ou aryle ;
R³ est choisi parmi un groupe alkyle, aryle ou Het ; et
P est choisi parmi les structures suivantes : 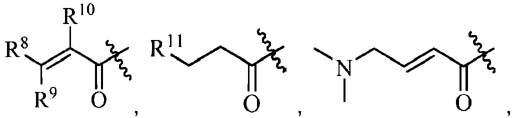
dans lesquelles R¹¹ est un groupe partant ou un ester activé, et le groupe partant est un halogène ; R⁸ , R⁹ et R¹⁰ sont chacun choisis indépendamment parmi l'hydrogène ou un groupe alkyle.

5. Composé selon la revendication 1, dans lequel le sel pharmaceutiquement acceptable comprend un sel d'addition d'acide formé par le composé de formule I avec les acides suivants : acide chlorhydrique, acide bromhydrique, acide sulfurique, acide phosphorique, acide méthanesulfonique, acide benzènesulfonique, acide p-toluènesulfonique, acide naphtalènesulfonique, acide citrique, acide tartrique, acide lactique, acide pyruvique, acide acétique, acide maléique ou acide succinique, acide fumarique, acide salicylique, acide phénylacétique, acide e et acide mandélique ; et comprend également un sel acide formé par le composé de formule I avec une base inorganique.

6. Composé selon la revendication 5, dans lequel le sel pharmaceutiquement acceptable comprend un sel de cation de métal alcalin, un sel de cation de métal alcalino-terreux et un sel de cation d'ammonium.

7. Composé selon la revendication 1, dans lequel le composé de formule I est l'un des composés suivants :

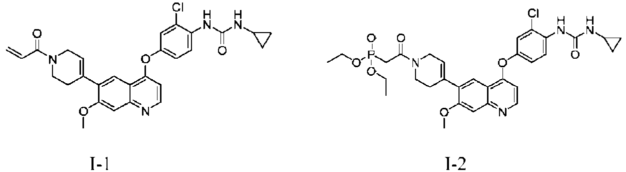

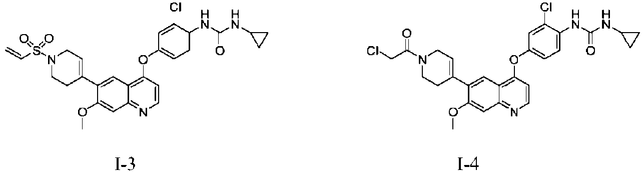

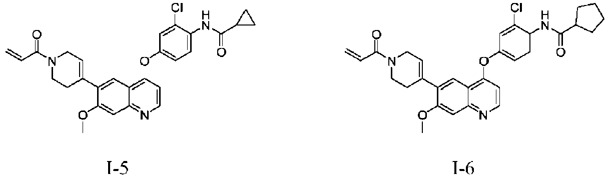

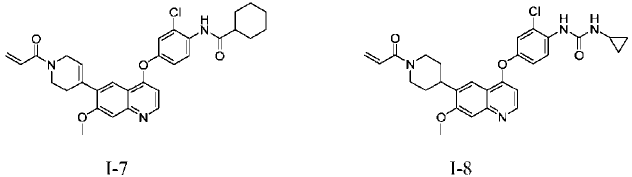

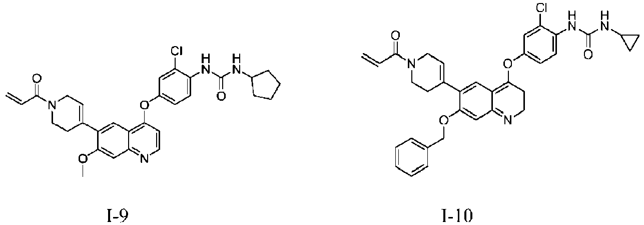

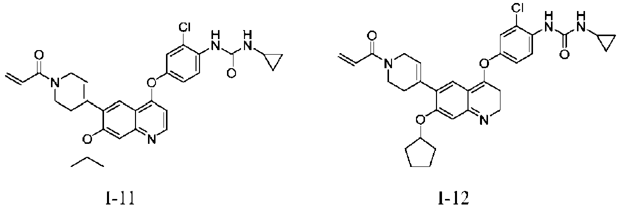

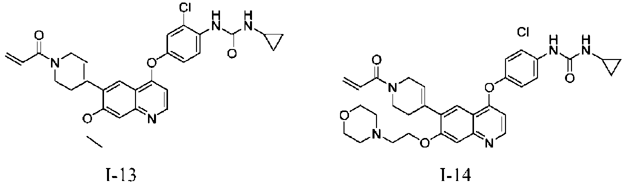

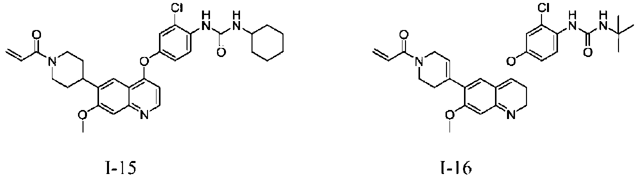

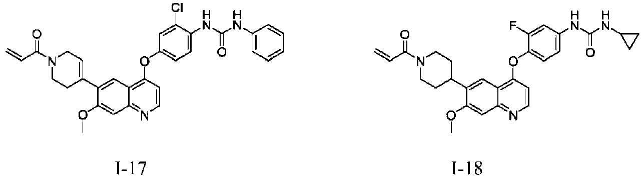

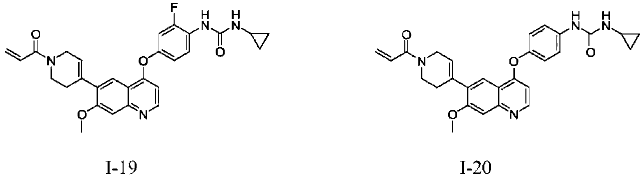

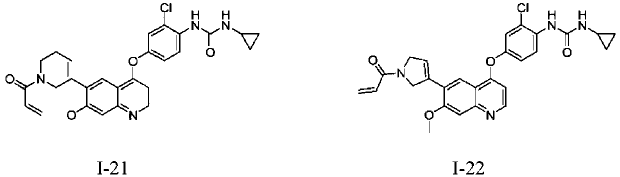

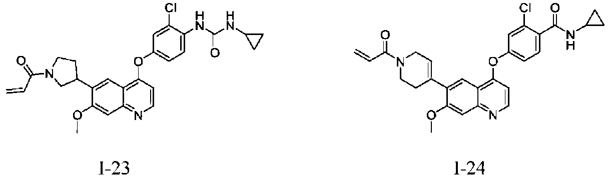

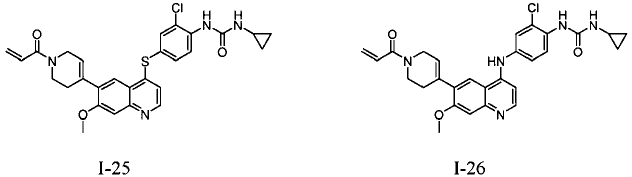

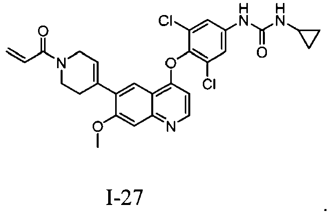

8. Composition pharmaceutique comprenant le composé ou son sel, sa forme cristalline, son stéréoisomère, son tautomère, son hydrate ou son solvate pharmaceutiquement acceptable selon la revendication 1, et un support ou excipient pharmaceutiquement acceptable.

9. Le composé selon la revendication 1 destiné à être utilisé dans la prévention et/ou le traitement de maladies liées au FGFR4 ; la maladie liée au FGFR4 étant choisie parmi l'hyperlipidémie ou le cancer ; et le cancer comprenant le cancer du poumon, le carcinome épidermoïde, le cancer de la vessie, le cancer de l'estomac, le cancer de l'ovaire, le cancer péritonéal, le cancer du sein, le carcinome canalaire du sein, le cancer de la tête et du cou, le cancer de l'endomètre, le cancer de l'utérus, le cancer du rectum, le cancer du foie, le cancer du rein, le cancer du bassinet rénal, le cancer de l'œsophage, l'adénocarcinome de l'œsophage, le gliome, le cancer de la prostate, le cancer de la thyroïde, le cancer de l'appareil reproducteur féminin, le carcinome in situ, le lymphome, la neurofibromatose, le cancer des os, le cancer de la peau, le cancer du cerveau, le cancer du côlon, le cancer des testicules, la tumeur stromale gastro-intestinale, le cancer de la bouche, le cancer du pharynx, le myélome multiple, la leucémie, le lymphome non hodgkinien, les adénomes villositaires colorectaux, les mélanomes, la tumeur cellulaire ou le sarcome, ou le syndrome myélodysplasique.
